# EUROPEAN PATENT APPLICATION

(11) **EP 3 272 286 A1**
(43) Date of publication of application: **24.01.2018**
(21) Application number: 17173448.6
(22) Date of filing: 30.05.2017
(51) Int. Cl.: A61B 6/00

(54) **RADIOGRAPHIC IMAGING APPARATUS, RADIATION IRRADIATION APPARATUS, AND METHOD OF CONTROLLING THE RADIOGRAPHIC IMAGING APPARATUS**

(30) Priority: 19.07.2016 KR 20160091570
(71) Applicant: Samsung Electronics Co., Ltd., Suwon-si, Gyeonggi-do (KR)
(72) Inventor: Kim, Seung Hoon, Yeongtong-gu, Suwon-si, Gyeonggi-do (KR); Yoon, Ja Woong, Seoul (KR)
(74) Representative: Gulde & Partner

(57) **Abstract**

Disclosed herein are a radiographic imaging apparatus, a radiation irradiation apparatus, and a method of controlling the radiographic imaging apparatus. The radiographic imaging apparatus comprises a radiation detector, a first support configured to enable the radiation detector to move in at least one direction of a up direction and a down direction, a first brake configured to stop the radiation detector at a plurality of positions and an image processor configured to synthesize a plurality of electrical signals output by the radiation detector at the plurality of positions to generate at least one radiation image.

## Description

### TECHNICAL FIELD

The present disclosure relates to a radiographic imaging apparatus, a radiation irradiation apparatus, and a method of controlling the radiographic imaging apparatus.

### BACKGROUND

A radiographic imaging apparatus is equipment to irradiate radiation such as X-rays on a subject to acquire an image about the inside of the subject so that a doctor or a security professional can visually examine the internal tissues, structure, objects, etc. of the subject. The subject may be a human body, an animal or plant, or baggage.

The radiographic imaging apparatus can acquire and provide images about the inside of a subject using a fact that radiation is absorbed in or transmitted through the internal material of a subject according to the characteristics (for example, density) of the internal material. Generally, the radiographic imaging apparatus can acquire radiation images by irradiating radiation on a subject, receiving the radiation transmitted through the subject, and then converting the radiation into an electrical signal.

The radiographic imaging apparatus is widely used in various industry fields, such as the health industry, the security system industry, or the construction industry.

### SUMMARY

To address the above-discussed deficiencies, it is a primary object to provide a radiographic imaging apparatus capable of moving a radiation emitter and/or a radiation detector to an appropriate scanning location, or appropriately adjusting the radiation irradiation direction of the radiation emitter to thereby easily and appropriately acquire a radiation image of high quality, a radiation irradiation apparatus, a radiation detection apparatus, and a method of controlling the radiographic imaging apparatus.

It is another aspect of the present disclosure to provide a radiographic imaging apparatus capable of guiding movements of a radiation emitter and a radiation detector upon stitching scanning to rapidly and easily locate the radiation emitter and the radiation detector at exact scanning locations, a radiation irradiation apparatus, a radiation detection apparatus, and a method of controlling the radiographic imaging apparatus.

Additional aspects of the disclosure will be set forth in part in the description which follows and, in part, will be obvious from the description, or can be learned by practice of the disclosure.

In accordance with one aspect of the present disclosure, a radiographic imaging apparatus includes a radiation detector, a first support configured to enable the radiation detector to move in at least one direction of a up direction and a down direction, a first brake configured to stop the radiation detector at a plurality of positions and an image processor configured to synthesize a plurality of electrical signals output by the radiation detector located at each position of the plurality of positions and to generate at least one radiation image.

The radiation detector can move in the down direction along the first support by the weight of the radiation detector.

The radiographic imaging apparatus can further include a first weight adder configured to apply an additional weight to the radiation detector.

The first weight adder can include at least one weight and a weight installing member in which the weight is installed.

The weight installing member can include at least one of a first weight installing member on which the weight is hung, a second weight installing member into which the weight is inserted, and a third weight installing member with which the weight is coupled by magnetism.

The radiation detector can move in the down direction along the first support by the additional weight.

The radiographic imaging apparatus can further include a second weight adder and a detector moving member configured to enable the radiation detector to move in the up direction along the first support by an additional weight applied by the second weight adder.

The detector moving member can include a rotary body, and further includes a rotation angle detector configured to sense an amount of rotation of the rotary body.

The radiographic imaging apparatus can further include a position detector configured to detect a position of the radiation detector when the radiation detector moves.

The first brake can stop the radiation detector at the plurality of positions according to the result of the detection by the position detector.

The radiographic imaging apparatus can further include a user interface configured to output a signal according to the result of comparison between the position of the radiation detector detected by the position detector and a scanning position of the radiation detector.

The first support can extend or be shortened such that the radiation detector is movable in at least one direction of the up direction and the down direction.

The radiographic imaging apparatus can further include a radiation emitter configured to irradiate radiation toward the radiation detector.

The radiographic imaging apparatus can further include a movable pulley connected to the radiation detector, a plurality of fixing pulleys configured to rotate to move the movable pulley in the up direction or in the down direction and a plurality of brakes configured to stop the plurality of fixing pulleys independently or dependently.

In accordance with one aspect of the present disclosure, a radiographic imaging apparatus can include a support, a radiation emitter connected to the support, and configured to rotate with respect to an axis of rotation to change an irradiation direction of radiation, a brake configured to stop the radiation emitter at a plurality of irradiation angles and a weight adder configured to apply an additional weight to a location spaced from the axis of rotation so that the radiation emitter rotates with respect to the axis of rotation.

The radiation emitter can rotate with respect to the axis of rotation to decide the irradiation direction of radiation, and wherein the radiation emitter has a center of gravity at the location spaced from the axis of rotation, and is rotatable with respect to the axis of rotation by the center of gravity.

The radiographic imaging apparatus can further include a second brake configured to stop the radiation emitter at the plurality of irradiation angles.

The second brake can stop the radiation emitter at an irradiation angle corresponding to a location at which a radiation detector is stopped by a first brake.

The radiographic imaging apparatus can further include a third weight adder configured to apply an additional weight to the radiation emitter at the location spaced from the axis of rotation, wherein the third weight adder is formed in at least one direction of a direction in which a radiation irradiation surface of the radiation emitter is located and a direction that is opposite to the direction in which the radiation irradiation surface of the radiation emitter is located, with respect to the axis of rotation.

In accordance with one aspect of the present disclosure, a method of controlling a radiographic imaging apparatus, can include disposing a radiation detector at a first position, moving the radiation detector in a up direction or in a down direction by an additional weight applied by a weight adder, when a brake is released, detecting a position of the radiation detector, operating the brake according to the position of the radiation detector and stopping the radiation detector at a second position by the operation of the brake.

Before undertaking the detailed description below, it may be advantageous to set forth definitions of certain words and phrases used throughout this patent document: the terms "include" and "comprise," as well as derivatives thereof, mean inclusion without limitation; the term "or," is inclusive, meaning and/or; the phrases "associated with" and "associated therewith," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, or the like; and the term "controller" means any device, system or part thereof that controls at least one operation, such a device may be implemented in hardware, firmware or software, or some combination of at least two of the same. It should be noted that the functionality associated with any particular controller may be centralized or distributed, whether locally or remotely. Definitions for certain words and phrases are provided throughout this patent document, those of ordinary skill in the art should understand that in many, if not most instances, such definitions apply to prior, as well as future uses of such defined words and phrases.

### BRIEF DESCRIPTION OF THE DRAWINGS

For a more complete understanding of the present disclosure and its advantages, reference is now made to the following description taken in conjunction with the accompanying drawings, in which like reference numerals represent like parts:
FIG. 1 shows a radiographic imaging apparatus according to an embodiment of the present disclosure;
FIG. 2 is a control block diagram of a radiation detection apparatus according to an embodiment of the present disclosure;
FIG. 3 is a perspective view of a radiation detection apparatus according to an embodiment of the present disclosure;
FIG. 4 is a perspective view of a weight adder according to another embodiment of the present disclosure;
FIG. 5 is a perspective view of a weight adder according to another embodiment of the present disclosure;
FIG. 6 is a first view for describing a first support according to a first embodiment of the present disclosure;
FIG. 7 is a second view for describing the first support according to the first embodiment of the present disclosure;
FIG. 8 is a third view for describing the first support according to the first embodiment of the present disclosure;
FIG. 9 is a first view for describing a first support according to a second embodiment of the present disclosure;
FIG. 10 illustrates a second weight adder according to an embodiment of the present disclosure;
FIG. 11 is a second view for describing the first support according to the second embodiment of the present disclosure;
FIG. 12 is a view for describing a first support according to a third embodiment of the present disclosure;
FIG. 13 is a view for describing a first support according to a fourth embodiment of the present disclosure;
FIG. 14 shows a first rotary body according to another embodiment of the present disclosure.
FIG. 15 is a first view for describing an example of driving of a first rotary body according to another embodiment of the present disclosure;
FIG. 16 is a second view for describing an example of driving of the first rotary body according to the other embodiment of the present disclosure;
FIG. 17 is a view for describing a method of detecting a position of a radiation detector, according to a first embodiment of the present disclosure;
FIG. 18 is a view for describing a method of detecting a position of a radiation detector, according to a second embodiment of the present disclosure;
FIG. 19 is a view for describing a method of detecting a position of a radiation detector, according to a third embodiment of the present disclosure;
FIG. 20 is a control block diagram of a radiation emitter according to an embodiment of the present disclosure;
FIG. 21 is a perspective view of a radiation emitter according to an embodiment of the present disclosure;
FIG. 22 is a view for describing an example of rotation of a radiation emitter according to an embodiment of the present disclosure;
FIG. 23 shows a third weight adder installed in a radiation emitter, according to a first embodiment of the present disclosure.
FIG. 24 is a view for describing an example of rotation of the radiation emitter in which the third weight adder according to the first embodiment of the present disclosure is installed;
FIG. 25 shows a second embodiment of a weight adder installed in a radiation emitter;
FIG. 26 is a view for describing an example of rotation of a radiation emitter to which the weight adder according to the second embodiment of the present disclosure is added;
FIG. 27 shows an embodiment of a fourth weight adder installed in a radiation emitter;
FIG. 28 is a view for describing an example in which a second support extends;
FIG. 29 is a block diagram of a control apparatus according to an embodiment of the present disclosure;
FIG. 30 shows an example of an acquired radiation image according to an embodiment of the present disclosure;
FIG. 31 shows an example of a synthesized radiation image according to an embodiment of the present disclosure;
FIG. 32 is a view for describing a process for setting an initial position of at least one of a radiation detector and a radiation emitter according to an embodiment of the present disclosure;
FIG. 33 is a view for describing a pattern of sound that is output from a sound output unit when at least one of a radiation detector and a radiation emitter approaches a scanning position;
FIG. 34 is a first flowchart illustrating a method of controlling a radiographic imaging apparatus, according to an embodiment of the present disclosure;
FIG. 35 is a first view for describing a process in which the radiographic imaging apparatus performs stitching scanning according to an embodiment of the present disclosure;
FIG. 36 is a second view for describing a process in which the radiographic imaging apparatus performs stitching scanning according to an embodiment of the present disclosure;
FIG. 37 is a third view for describing a process in which the radiographic imaging apparatus performs stitching scanning according to an embodiment of the present disclosure;
FIG. 38 is a second flow chart illustrating a method of controlling a radiographic imaging apparatus, according to an embodiment of the present disclosure; and
FIG. 39 is a flowchart illustrating a method of setting the initial positions of a radiation emitter and a radiation detector, according to an embodiment of the present disclosure, in a method of controlling a radiographic imaging apparatus.

### DETAILED DESCRIPTION

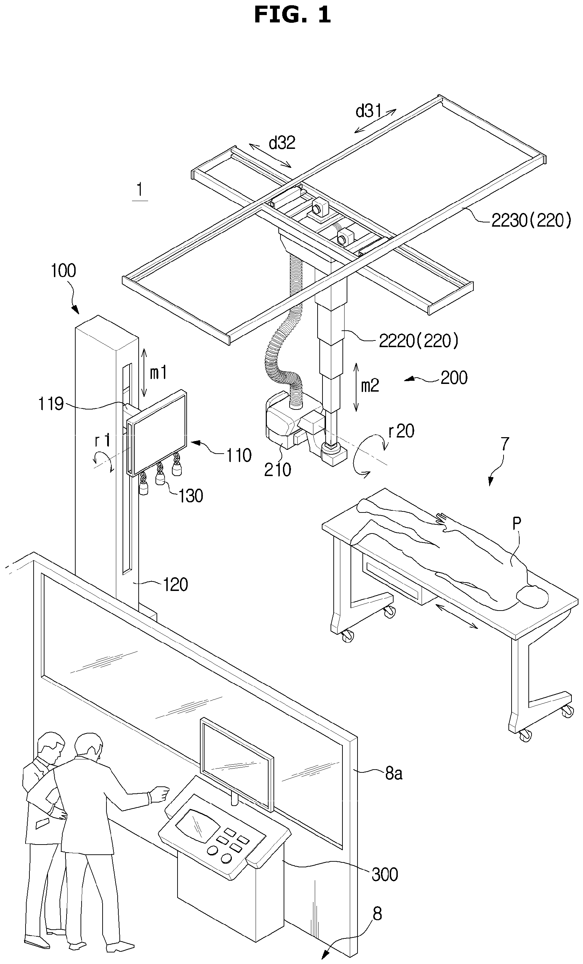

Figures 1 through 39, discussed below, and the various embodiments used to describe the principles of the present disclosure in this patent document are by way of illustration only and should not be construed in any way to limit the scope of the disclosure. Those skilled in the art will understand that the principles of the present disclosure may be implemented in any suitably arranged electronic device.

Hereinafter, a radiographic imaging apparatus, a radiation irradiation apparatus, and a radiation detection apparatus according to embodiments of the present disclosure will be described in detail with reference to FIGS. 1 to 33.

FIG. 1 shows a radiographic imaging apparatus according to an embodiment of the present disclosure.

Referring to FIG. 1, a radiographic imaging apparatus 1 according to an embodiment of the present disclosure includes: a radiation irradiation apparatus 200 configured to irradiate radiation such as X-rays; a radiation detection apparatus 100 configured to receive and detect the radiation irradiated by the radiation irradiation apparatus 200; and a control apparatus 300 configured to control overall operations of the radiographic imaging apparatus 1. The control apparatus 300 generates a radiation image corresponding to the radiation received by the radiation detection apparatus 100, and receives commands from a user or provide the user with various information.

At least one of the radiation detection apparatus 100 and the radiation irradiation apparatus 200 can be communicatively connected to the control apparatus 300 to receive various control signals from the control apparatus 300 or to transfer acquired data to the control apparatus 300. Also, the radiation irradiation apparatus 200 and the radiation detection apparatus 100 can be configured to transmit and receive electrical signals to and from each other, according to a designer's selection.

At least two of the radiation detection apparatus 100, the radiation irradiation apparatus 200 and the control apparatus 300 can be communicatively connected each other using at least one of a wired communication unit and a wireless communication unit.

The wired communication unit can be implemented by using a cable, such as a pair cable, a coaxial cable, an optical fiber cable or an Ethernet cable.

The wireless communication unit can be implemented based on at least one of a local area network technology and mobile network technology. The local area network technology can include at least one of wireless LAN, Wi-Fi, Bluetooth, zigbee, CAN communication system, Wi-Fi Direct, ultra wideband communication system, IrDA, infrared Data Association, Bluetooth Low Energy, or Near Field Communication (NFC). The mobile communication technology can include at least one of wireless communication technology that is implemented by using a variety of mobile communication stands, such as 3GPP-based communication system, 3GPP2-based communication system, or Wi MAX-based communication system.

The radiation detection apparatus 100 and the radiation irradiation apparatus 200 can be placed in an examination room 7 in order to prevent an examiner from being exposed to radiation, and the control apparatus 300 can be placed in separate space 8 partitioned by a baffle wall 8a from the examination room 7.

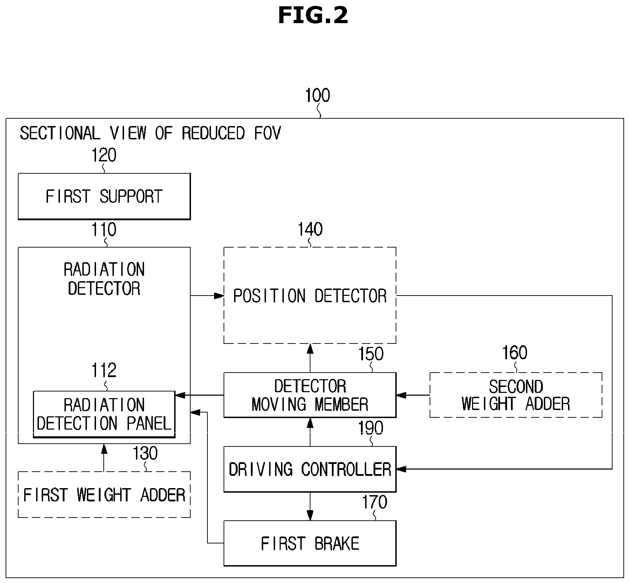

Hereinafter, the radiation detection apparatus 100 will be described in more detail herein below with respect to FIG. 2.

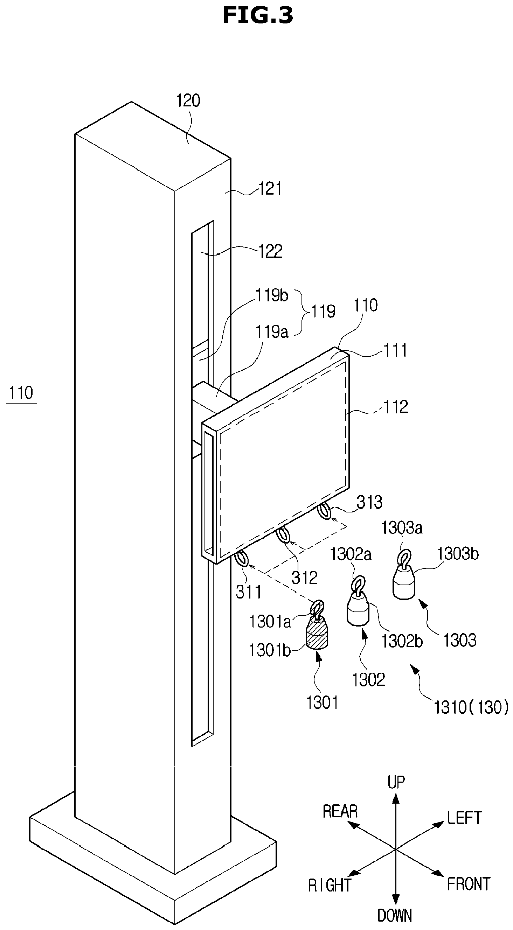

FIG. 2 is a control block diagram of a radiation detection apparatus according to an embodiment of the present disclosure, and FIG. 3 is a perspective view of a radiation detection apparatus according to an embodiment of the present disclosure.

Referring to FIGS. 2 and 3, the radiation detection apparatus 100 can include a radiation detector 110 configured to detect radiation, a first support 120 along which the radiation detector 110 moves, a detector moving member 150 configured to move the radiation detector 110, and a first brake 170 configured to stop the radiation detector 110 at a specific location.

Also, the radiation detection apparatus 100 can further include at least one of a first weight adder 130 to add an additional weight to the radiation detector 110, and a second weight adder 160 to apply a force to the radiation detector 110 in a direction that is opposite to the direction of gravity.

As shown in FIGS. 2 and 3, the radiation detector 110 can include a radiation detection panel 112 to receive radiation irradiated from a radiation emitter 210 (as shown in FIG. 1) and to convert the received radiation into an electrical signal, and a radiation detector housing 111 in which the radiation detection panel 112 and the related components are installed. The radiation incident to the radiation detection panel 112 can be radiation transmitted through an object.

The radiation detection panel 112 can convert the received radiation into an electrical signal using a direct conversion method or an indirect conversion method. The electrical signal corresponding to the received radiation can be transferred to an image processor 301 of the control apparatus 300. The radiation detector 110 can further include an anti-scattering grid to guide radiation transmitted through an object and then scattered to be appropriately incident to the radiation detection panel 112.

The radiation detector housing 111 can fix the radiation detection panel 112 stably, wherein the front surface of the radiation detector housing 111 faces the outside, and the rear surface of the radiation detector housing 111 faces the first support 120. In this case, the front surface of the radiation detector housing 111 faces the radiation irradiation apparatus 200.

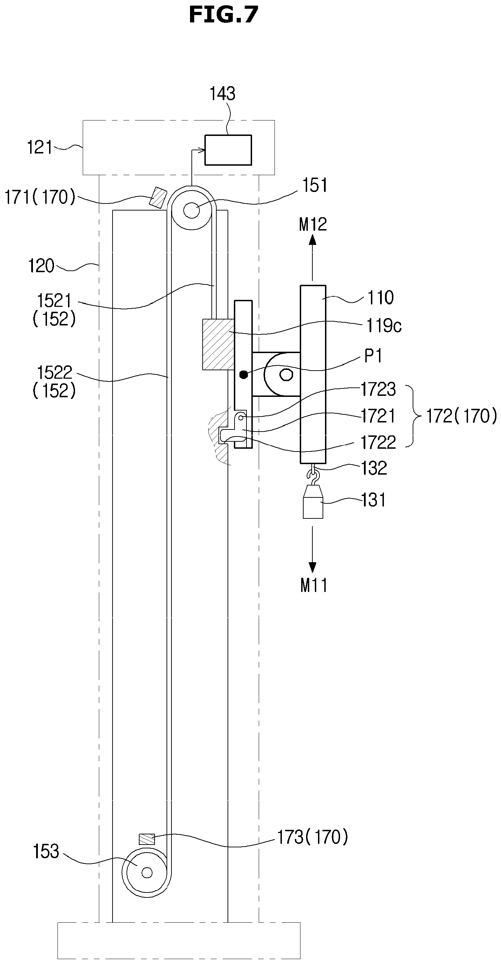

In the rear surface of the radiation detector housing 111, a moving body coupling member 119 can be formed to be coupled with and fixed at a moving body (152 of FIG. 7) that is movable in the inside of the first support 120. The moving body coupling member 119 can connect the radiation detector housing 111 to the moving body 152 so as to move the moving body 152 correspondingly when the radiation detector housing 111 moves upward or downward, and to move the radiation detector housing 111 correspondingly when the moving body 152 moves.

According to an embodiment, the moving body coupling member 119 can include a first coupling part 119a passing through an opening 122 formed in one side of the first support 120, and inserted into the inside of the first support 120, a second coupling part 119b connected to the first coupling part 119a and configured to guide the radiation detector 110 to move along the first support 120, and a third coupling part (119c shown in FIG. 7) with which the moving body 152 is coupled. According to an embodiment, the first coupling part 119a can further include a rotation shaft member to tilt the radiation detector 110 to a predetermined angle. The radiation detector 110 can be rotated in a predetermined rotation direction r1 by the rotation shaft member, so that a direction which the front surface of the radiation detector 110 faces can change.

The radiation detector panel 112, the radiation detector housing 111, and components (for example, various kinds of control boards) installed in the radiation detector housing 111 have their own weights, and the radiation detector 110 also has its own weight. The weight of the radiation detector 110 can move the radiation detector 110 in the direction of gravity unless the first brake 170 operates.

In the radiation detector housing 111, the first weight adder 130 can be provided to add an additional weight to the radiation detector 110. Due to the additional weight provided by the first weight adder 130, the radiation detector 110 can move in the direction of gravity with a greater force.

According to an embodiment, the first weight adder 130 can be installed at various locations of the radiation detector housing 111. For example, the first weight adder 130 can be disposed in the lower end of the radiation detector housing 111, as shown in FIG. 3. Also, the first weight adder 130 can be installed in different locations of the radiation detector housing 111, for example, in the upper end, front surface, lateral sides, or rear surface of the radiation detector housing 111, according to the designer's selection. The first weight adder 130 can be implemented in various shapes 1310, 1320, and 1330, as shown in FIGS. 3, 4, and 5.

Referring to FIG. 3, a first weight adder 1310 according to a first embodiment of the present disclosure can include one or more weights 1301, 1302, and 1303, and one or more weight installing members 1311, 1312, and 1313 on which the one or more weights1301, 1302, and 1303 are hung.

According to an embodiment, the weights 1301, 1302, and 1303 can include one or more weight bodies 1301b, 1302b, and 1303b each having a predetermined weight, and one or more hooks 1301a, 1302a, and 1303a formed on the upper ends of the weight bodies 1301b, 1302b, and 1303b. The first weight installing members 1311, 1312, and 1313 can be formed in the lower end of the radiation detector housing 111, and include one or more loops by which the hooks 1301a, 1302a, and 1303a can be caught. The hooks 1301a, 1302a, and 1303a can be caught by the first weight installing members 1311, 1312, and 1313 so that the weights 1301, 1302, and 1303 can be coupled with the radiation detector 110, and accordingly, the weights 1301, 1302, and 1303 can add an additional weight to the radiation detector 110.

According to another embodiment, contrary to the above-described embodiment, the weights 1301, 1302, and 1303 can include loops, and the first weight installing members 1311, 1312, and 1313 can include hooks by which the loops of the weights 1301, 1302, and 1303 can be caught.

According to another embodiment, all of the first weight installing members 1311, 1312, and 1313 and the weights 1301, 1302, and 1303 can include hooks that can be caught by each other.

According to a user's need (for example, a radiological technologist's need), only one weight 1302 can be hung on the first weight installing member 1311 of the first weight installing members 1311, 1312, and 1313, or two weights 1301 and 1302 can be hung on the first weight installing members 1311 and 1312 of the first weight installing members 1311, 1312, and 1313. Also, the weights 1301, 1302, and 1303 can be hung on the first weight installing members 1311, 1312, and 1313, respectively. As such, by changing the number of the weights 1301, 1302, and 1303 to be hung on the first weight installing members 1311, 1312, and 1313, the user can add various weights to the radiation detector 110.

The weights 1301, 1302, and 1303 can have the same weight, or different weights.

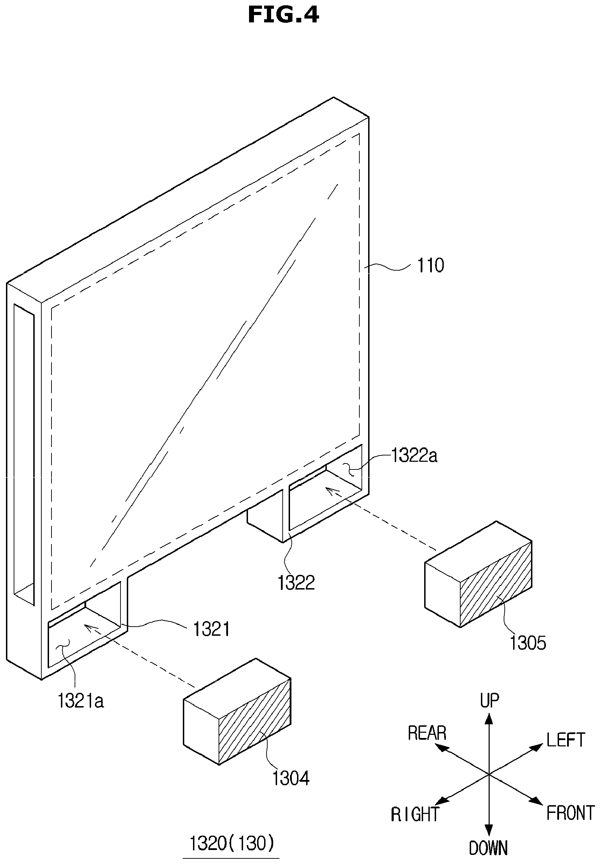

FIG. 4 is a perspective view of a weight adder according to another embodiment of the present disclosure.

Referring to FIG. 4, a first weight adder 1320 according to a second embodiment of the present disclosure can include one or more weights 1304 and 1305, and one or more second weight installing members 1321 and 1322 in which the one or more weights 1304 and 1305 can be inserted.

Each of the weights 1304 and 1305 can have a predetermined shape, and for example, each of the weights 1304 and 1305 can be in the shape of a cuboid, a cylinder, a sphere, or another geometrical structure. In this case, the weights 1304 and 1305 can have shapes corresponding to resting spaces 1321 a and 1322a of the second weight installing members 1321 and 1322.

The second weight installing members 1321 and 1322 can have shapes in which the weights 1304 and 1305 having the predetermined shape can be inserted. For example, the second weight installing members 1321 and 1322 can include a frame having a surface in which the resting spaces 1321a and 1322a into which the weights 1304 and 1305 can be inserted are formed, wherein the frame can be in the shape of a cuboid, a cylinder, a sphere, or another geometrical structure. The weights 1304 and 1305 can be inserted and installed in the resting spaces 1321 a and 1322a through openings.

According to embodiments, there can be provided a second weight installing member or two second weight installing members. Or, there can be provided three weight installing members or more. In the front portions of the second weight installing members 1321 and 1322, covers (not shown) for covering the resting spaces 1321 a and 1322a can be further provided as necessary. The covers can expose the openings and the resting spaces 1321a and 1322a to the outside or close them from the outside, according to a user's selection.

According to embodiments, the second weight installing members 1321 and 1322 can be disposed in the lower end of the radiation detector housing 111, as shown in FIG. 4, or in any one(s) of the rear surface, front surface, lateral sides, or upper end of the radiation detector housing 111, according to a designer's selection.

If the weights 1304 and 1305 are inserted into the second weight installing members 1321 and 1322 and rested in the second weight installing members 1321 and 1322, the weights 1304 and 1305 can add additional weights to the radiation detector 110, and the radiation detector 110 can move in the direction of gravity due to the applied weights.

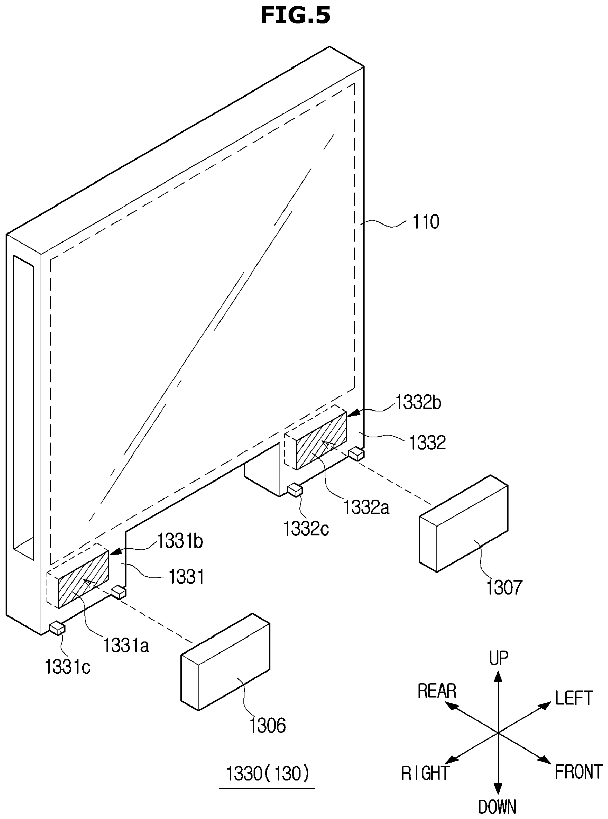

FIG. 5 is a perspective view of a weight adder according to another embodiment of the present disclosure.

Referring to FIG. 5, a first weight adder 1330 according to a third embodiment of the present disclosure can include one or more weights 1306 and 1307, and one or more third weight installing members 1331 and 1332 on which the one or more weights 1306 and 1307 can be attached.

The weights 1306 and 1307 can be implemented with a magnetic substance, such as iron, a permanent magnet, or an electromagnet, so as to be able to be attached on the third weight installing members 1331 and 1332 using magnetism.

The third weight installing members 1331 and 1332 can enable the weights 1306 and 1307 to be attached on the radiation detector housing 111 using magnetism. For example, the third weight installing members 1331 and 1332 can include contact surfaces 1331b and 1332b provided to expose permanent magnets or electromagnets 1331 a and 1332a and magnetism emitted from the permanent magnets or electromagnets 1331 a and 1332a to the outside, and the weights 1306 and 1307 can be attached on the third weight installing members 1331 and 1332 by magnetism.

According to an embodiment, in order to prevent the weights 1306 and 1307 attached on the contact surfaces 1331 a and 1332a from escaping from the third weight installing members 1331 and 1332, the third weight installing members 1331 and 1332 can further include one or more weight supports 1331c and 1332c. According to an embodiment, the weight supports 1331c and 1332c can be protrusions protruding in the front direction of the radiation detector 110. Each protrusion can be, for example, in the shape of a cuboid or a "L"-shaped structure. Also, various means for preventing the weights 1306 and 1307 from escaping from the third weight installing members 1331 and 1332 can be used as the weight supports 1331 c and 1332c.

According to embodiments, the third weight installing members 1331 and 1332 can be disposed in the lower end of the radiation detector housing 111, or in the rear surface, front surface, lateral sides, or upper end of the radiation detector housing 111.

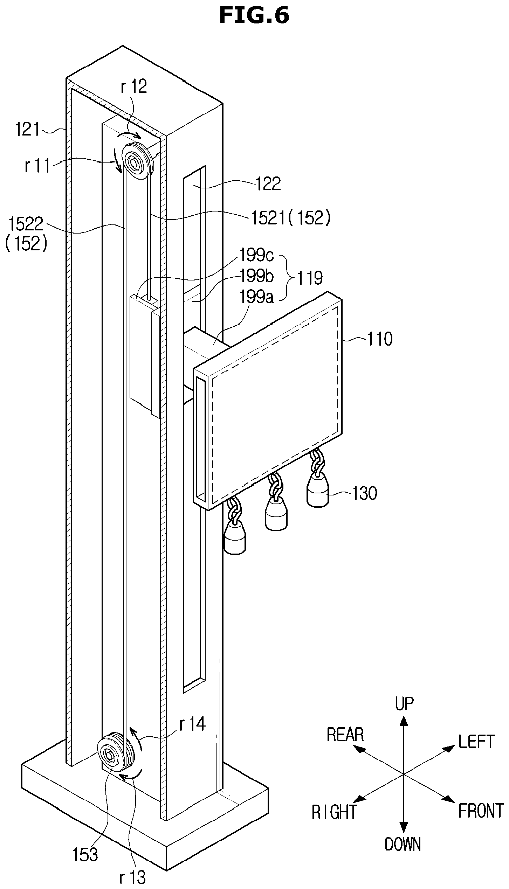

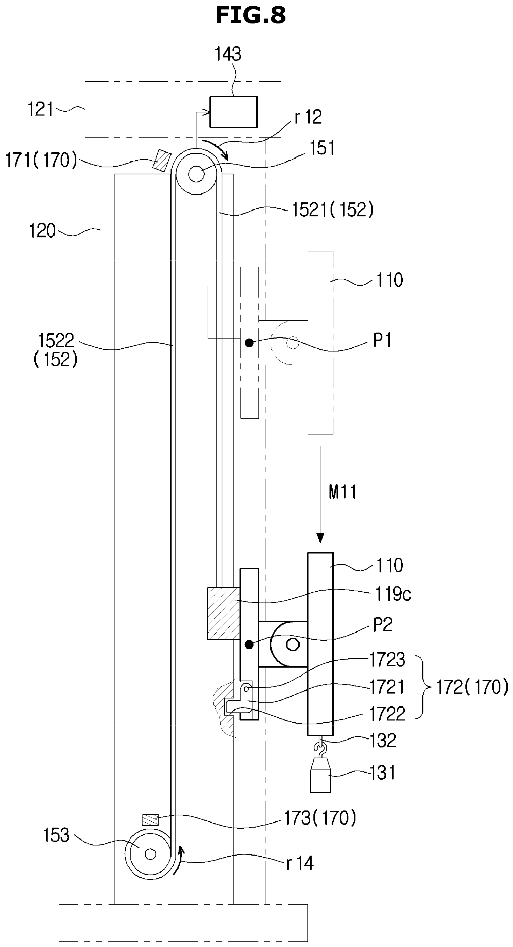

FIG. 6 is a first view for describing a first support according to a first embodiment of the present disclosure, FIG. 7 is a second view for describing the first support according to the first embodiment of the present disclosure, and FIG. 8 is a third view for describing the first support according to the first embodiment of the present disclosure.

The first support 120 can support the radiation detector 110, and guide the radiation detector 110 to move upward or downward. According to an embodiment, the first support 120 can be provided in the shape of a stand, as shown in FIGS. 1 and 3. According to another embodiment, the first support 120 can be in the shape of a post frame (see 2220 of FIG. 1). In this case, the first support 120 can extend or be shortened to guide the radiation detector 110 to move upward or downward. Also, the first support 120 can be fabricated in various shapes that can be considered by a designer.

According to an embodiment, the first support 120 can include a first support housing 121, and a guide hole 122 formed in one side of the first support housing 121 and configured to guide movement of the radiation detector 110. The first coupling part 119a of the moving body coupling member 119 can pass through the guide hole 122.

Referring to FIGS. 6, 7, and 8, in the inside of the first support housing 121, the detector moving member 150 can be provided to move the radiation detector 110 in at least one direction M11, M12.

The detector moving member 150 can include one or more rotary bodies 151 and 153, and a moving body 152 coupled with the third coupling part 119c and configured to move the radiation detector 110 according to rotation of at least one of the rotary bodies 151 and 153.

The rotary bodies 151 and 153 can be implemented as pulleys, toothed wheels, or the like. The rotary bodies 151 and 153 can rotate in a predetermined direction r11, r12, r13, or r14 to move the moving body 152 wound on the rotary bodies 151 and 153.

According to an embodiment, the rotary bodies 151 and 153 can include a first rotary body 151 and a second rotary body 153.

The first rotary body 151 can be disposed in the upper portion of the first support 120, and the moving body 152 can be caught by the upper portion of the first rotary body 151. The second rotary body 153 can be disposed in the lower portion of the first support 120, and the moving body 152 can be wound on or released from the second rotary body 153 according to rotation of the second rotary body 153.

At least one of the first and second rotary bodies 151 and 153 can be interlocked with the moving body 152. In other words, if at least one of the first and second rotary bodies 151 and 153 rotates in a predetermined direction r11, r12, r13, or r14, the moving body 152 can also move in the predetermined direction r11, r12, r13, or r14 accordingly, or if the moving body 152 moves in a predetermined direction, at least one of the first and second rotary bodies 151 and 152 can rotate in a direction corresponding to the moving direction of the moving body 152. According to an embodiment, the first support 120 can include a plurality of second rotary bodies 153.

In FIGS. 6, 7, and 8, two rotary bodies 151 and 153 are shown; however, the number of the rotary bodies 151 and 153 is not limited to two. That is, a single rotary body or three or more rotary bodies can be installed in the first support 120, according to a designer's selection.

The moving body 152 can be wound on the first and second rotary bodies 151 and 153, wherein one end of the moving body 152 can be connected to the third coupling part 119c, and the other end of the moving body 152 can be attached on the second rotary body 153 in such a way not to escape from the second rotary body 153. The moving body 152 can be implemented as means that can be wound on the first and second rotary bodies 151 and 153 and can move in correspondence to rotation of the first and second rotary bodies 151 and 153. For example, the moving body 152 can be implemented as a cable, a wire, a belt, a chain, or the like. The moving body 152 can be implemented according to the kind of the rotary bodies 151 and 153. For example, if the rotary bodies 151 and 153 are toothed wheels, the moving body 152 can be implemented as a chain or a belt having a groove that can be caught by the teeth of the rotary bodies 151 and 153.

The first brake 170 can stop the radiation detector 110 at a specific position. The first brake 170 can operate according to a control signal received from a driving controller 190 to stop the radiation detector 110 a specific position.

According to an embodiment, the first brake 170 can include at least one of a first rotary body brake 171 disposed around the first rotary body 151, a side brake 172 disposed on one side of the first support housing 121, and a second rotary body brake 173 disposed around the second rotary body 153.

The first rotary body brake 171 can be disposed around the first rotary body 151, and stop the first rotary body 151. If the first rotary body 151 stops rotating, the moving body 152 can also stop moving, and accordingly, the radiation detector 110 can also stop moving. The first rotary body brake 171 can apply a friction force to the first rotary body 151 using means such as a pad to stop the first rotary body 151, or the first rotary body brake 171 can stop the first rotary body 151 using a magnetic field. Or, the first rotary body brake 171 can stop the first rotary body 151 using the pressure of air or fluid. Also, various braking means for stopping the first rotary body 151 can be used as the first rotary body brake 171.

The side brake 172 can include an insertion protrusion 1721 formed in the radiation detector 110, at least one insertion hole 1722 formed in the outer surface of the first support housing 121, and a rotation shaft member 1723 formed in the radiation detector 110 and configured to enable the insertion protrusion 1721 to rotate within a predetermined angle range with respect to a predetermined axis.

The side brake 172 can insert the insertion protrusion 1721 into the at least one insertion hole 1722 to stop the radiation detector 110. In this case, the insertion protrusion 1721 can rotate at a predetermined angle in the direction of the first support housing 121 by the rotation shaft member 1723 to be inserted into the insertion hole 1722, wherein the rotation shaft member 1723 can be rotated by a driver such as a motor. The at least one insertion hole 1722 can be formed with a predetermined pattern in the outer surface of the first support housing 121, and the radiation detector 110 can stop at different locations according to the number and location of the at least one insertion hole 1722.

According to another embodiment, the insertion protrusion 1721 and the rotation shaft member 1723 can be formed in the first support housing 121, and the insertion hole 1722 can be formed in the radiation detector 110.

The second rotary body brake 173 can be disposed around the second rotary body 153, and can stop the second rotary body 153 in the same manner as the first rotary body brake 171 or in a different manner from the first rotary body brake 171. If the second rotary body 153 stops, the moving body 152 can stop moving, and accordingly, the radiation detector 110 can also stop moving. The second rotary body brake 173 can apply a friction force to stop the second rotary body 153, or can use a magnetic field to stop the second rotary body 153. Also, the second rotary body brake 173 can use various braking means for stopping the rotary body 153.

Hereinafter, a process of moving the radiation detector 110 downward by an additional weight applied by the first weight adder 130 will be described.

As shown in FIGS. 7 and 8, when the radiation detector 110 is located at a first point p1, a user can install a weight 131 in a weight installing member 132 of the radiation detector 110. In this case, an additional weight can be applied to the radiation detector 110, and due to the weight of the radiation detector 110 and the additional weight, a force in the direction M11 of gravity can be applied to the radiation detector 110. The force applied to the radiation detector 110 can be transferred to the moving body 152 so that tension can be generated in the moving body 152. The tension applied to the moving body 152 by the weight of the radiation detector 110 and the additional weight can exceed a friction force between the rotation shaft members of the first and second rotary bodies 151 and 153 and the surface on which the rotation shaft members are located, and accordingly, the moving body 152 can move in the direction of the applied force. The first rotary body 152 and the second rotary body 153 can rotate in the directions r12 and r14 according to the direction of the force applied to the moving body 152. In this case, a portion 1521 of the moving body 152 connected to the third coupling part 119c can extend, and the other portion 1522 of the moving body 152 can be shortened. Accordingly, the radiation detector 110 can move in the direction M11 of gravity, that is, in a down direction M11.

When the radiation detector 110 moves, the driving controller 190 can generate a brake signal according to a predetermined setting, and the brake signal can be transferred to the first brake 170. The first brake 170 can stop the rotary bodies 151 and 153, or can cause the insertion protrusion 1721 formed in the radiation detector 110 to be inserted into the insertion hole 1722, according to the brake signal, so as to stop the radiation detector 110 at a desired second point p2. Accordingly, the radiation detector 110 can move smoothly in the down direction M11, and stop at the desired second position p2, without a user's manual manipulation.

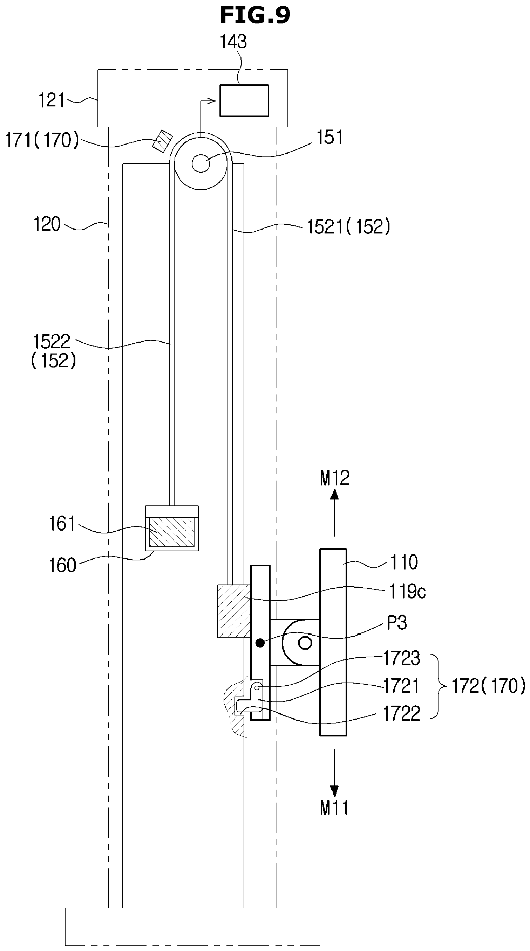

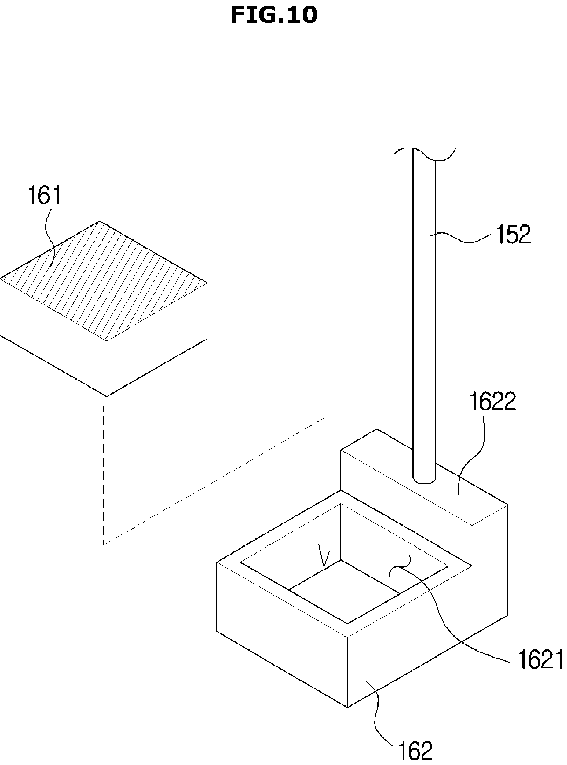

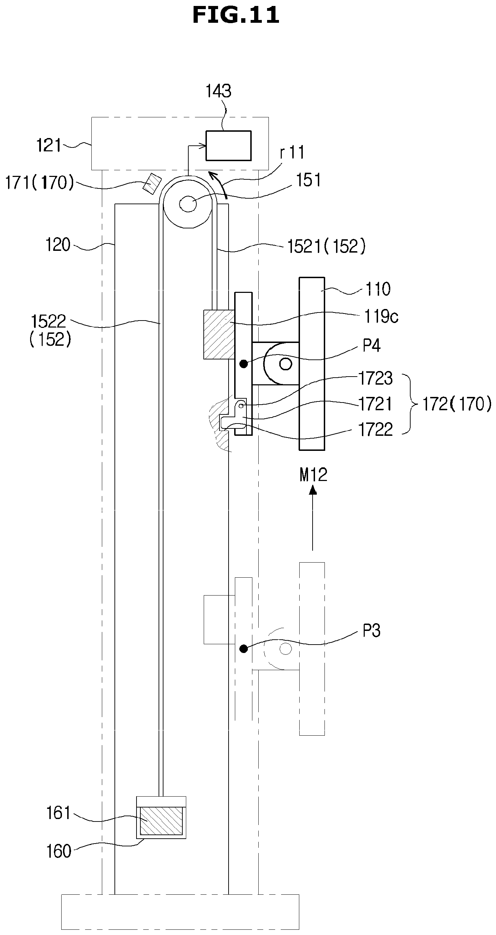

FIG. 9 is a first view for describing a first support according to a second embodiment of the present disclosure, FIG. 10 shows a second weight adder, and FIG. 11 is a second view for describing the first support according to the second embodiment of the present disclosure.

As shown in FIG. 9, in the inside of the first support 120 according to a second embodiment of the present disclosure, the detector moving member 150 and a brake 170 can be installed. Also, a second weight adder 160 can be further installed in the inside and/or outside of the first support 120.

The detector moving member 150 can include a rotary body 151 and a moving body 152.
The rotary body 151 can be disposed in the upper end of the first support 120, and the moving body 152 can be movable on the upper portion of the rotary body 151. The rotary body 151 can be implemented as a pulley, a toothed wheel, or the like. The rotary body 151 can be interlocked with the moving body 152 to rotate according to movement of the moving body 152.

The moving body 152 can be wound on the rotary body 151 while contacting the rotary body 151. The moving body 152 can be implemented as a cable, a wire, a belt, a chain, or the like. One end of the moving body 152 can be connected to the moving body coupling member 119 to couple the moving body 152 with the radiation detector 110, and the other end of the moving body 152 can be coupled with the second weight adder 160 to apply a force to the moving body coupling member 119 in a up direction M12.

The brake 170 can be disposed around the rotary body 151, and include at least one of a first brake 171 to stop the rotary body 151 and a side brake 172 disposed in one side of the first support housing 121. Operations of the first brake 171 and the side brake 172 have been described above, and accordingly further descriptions thereof will be omitted. The second weight adder 160 can add a predetermined weight to the other end of the moving body 152.

According to an embodiment, the second weight adder 160 can include, as shown in FIG. 10, a weight 161 and an installing member 162 in which the weight 161 is inserted.

The weight 161 can have a predetermined weight, and can have a shape that can be inserted in resting space 1621 of the installing member 162. The weight 161 can include a predetermined gripping part (not shown) to enable a user to easily insert the weight 161 into the resting space 1621 or easily pick the weight 161 out of the resting space 1621, as necessary.

The installing member 162 can include the resting space 1621 in which the weight 161 is inserted, and a main body 1622 in which the resting space 1621 is formed. The resting space 1621 can have an appropriate shape which one or more weights 161 can be easily inserted into or picked out of. For example, if the weight 161 is in the shape of a cuboid, the insertion space 1611 can also be in the shape of a cuboid to correspond to the shape of the weight 161. The installing member 162 can further include a cover (not shown) to open or close the resting space 1621, as necessary.

If the user inserts the weight 161 into the installing member 162 to install the weight 161 in the resting member 162, the weight 161 can apply a force (that is, tension) to the moving body 152 in the down direction M11. Meanwhile, the application direction of a force can change by the rotary body 151 so that a force in the up direction M12 can be applied to the moving body coupling member 119. The tension applied to the moving body 152 by the weight 161 can exceed a friction force between the rotary body 151 and the surface on which the rotary body 151 is located, so that the second weight adder 160 can move in the down direction as shown in FIG. 11, and the moving body coupling member 119 can move in the up direction M12. In this case, the radiation detector 110 coupled with the moving body coupling member 119 can also move in the up direction M12 from a third point p3.

While the radiation detector 110 moves, the driving controller 190 can generate a brake signal according to a predetermined setting, and the brake signal can be transferred to the first brake 170. The first brake 170 can stop the rotary body 151, or cause the insertion protrusion 1721 formed in the radiation detector 110 to be inserted into the insertion hole 1722 so as to stop the radiation detector 110 at a fourth point p4 that is higher than the third point p3. Accordingly, the radiation detector 110 can move smoothly in the up direction M12 and be located at a desired position, without the user's manipulation.

As such, since the radiation detector 110 can automatically move smoothly by the weight of the radiation detector 110 and the first weight adder 130 or by the second weight adder 160, the radiation detection apparatus 100 does not require any additional component for moving the radiation detector 110 for stitching scanning, which contributes to simplification in structure and a reduction of manufacturing costs.

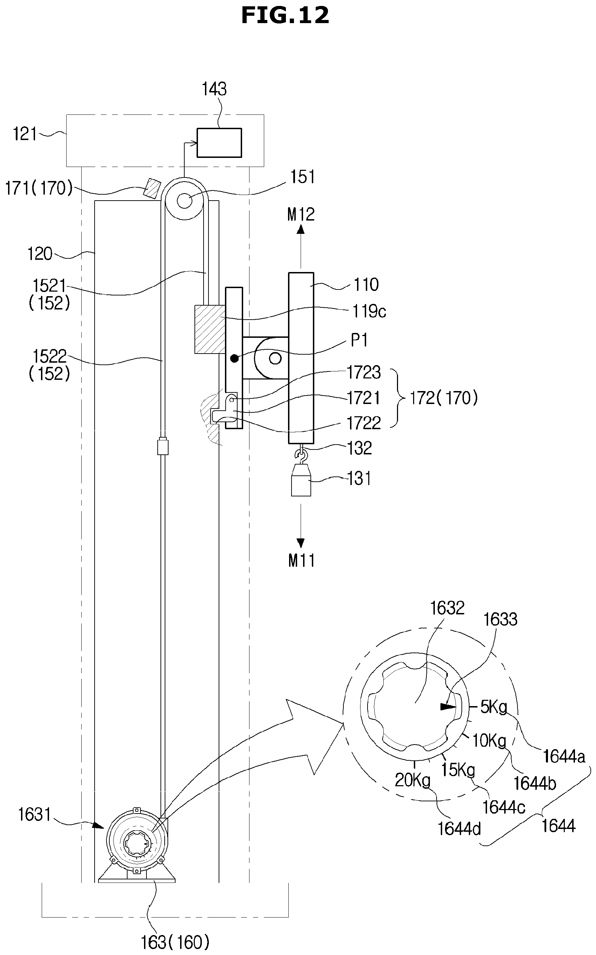

FIG. 12 is a view for describing a first support according to a third embodiment of the present disclosure.

As shown in FIG. 12, in the inside of the first support 120, according to a third embodiment of the present disclosure, there is included the detector moving member 150 that moves the radiation detector 110, the brake 170 that stops the radiation detector 110 at one or more positions, and the second weight adder 160 that applies a force to the radiation detector 110 in a direction that is opposite to the direction of gravity. The second weight adder 160 can be implemented as a spring balancer 163.

The detector moving member 150 can be disposed in the upper end of the first support 120, and include the rotary body 151 along which the moving body 152 can move, and the moving body 152 to move according to rotation of the rotary body 151 to thus move the radiation detector 110.

One end of the moving body 152 can be connected to the radiation detector 110 through the moving body coupling member 119, and the other end of the moving body 152 can be connected to the spring balancer 163.

The brake 170 can be disposed around the rotary body 151, and can include at least one of the first brake 171 to stop the rotary body 151 and the side brake 172 disposed in one side of the first support housing 121.

The detector moving member 150 and the brake 170 have been described above, and accordingly, further descriptions thereof will be omitted.

The spring balancer 163 means a device for maintaining the tension of a cable or a wire constant using an elastic body therein. The elastic body of the spring balancer 163 can be implemented as, for example, a spring coil, and also the spring balancer 163 can be used as the second weight adder 160.

The spring balancer 163 can include a tension adjusting part 1631 to adjust tension that is applied to the moving body 152, on its at least one outer surface, as shown in FIG. 12. The tension adjusting part 1631 can be mechanically or physically implemented. For example, the tension adjusting part 1631 can be implemented as a physical button, a knob, a trackball, a stick, a lever, a thread groove, a touch pad, or a touch screen. For example, if the tension adjusting part 1631 is implemented as a knob, the user can rotate the knob to adjust a weight or a force that the spring balancer 163 applies to the moving body 152. According to another example, if the tension adjusting part 1631 is implemented as a thread groove, the user can rotate a screw using a driver corresponding to the thread groove to thus adjust a weight or force that the spring balancer 163 applies.

If the tension adjusting part 1631 is implemented as a knob, at least one selection item 1644 can be formed on a surface around the knob, using printing, etc., and an indicator 1632 for indicating a selected selection item 1644 can be provided on the outer surface of the knob.

The selection item 1644 can represent, for example, the magnitude of tension. In this case, the selection item 1644 can be represented by further including units of weight (kg) or the magnitude (N) of force. Also, according to another example, the selection item 1644 can represent the direction of a force that is applied to the radiation detector 110. For example, the selection item 1644 can be represented using terms, such as "upward", "balance", and "downward". Also, the selection item 1644 can be formed in various kinds and formats on the surface around the knob, according to a designer's selection.

The indicator 1632 can indicate a selection item (for example, a first selection item 1644a) of a plurality of selection items 1644a to 1644d, representing a current magnitude of tension or a direction of a force applied to the radiation detector 110. The user can rotate the knob with reference to the plurality of selection items 1644a to 1644d to thereby adjust a weight or force which the spring balancer 163 applies to the moving body 152.

If the spring balancer 163 is used, the user can easily adjust a force or weight that is applied to the other end of the moving body 152. If the brake 170 is released as shown in FIGS. 7, 8, 9, and 11, the radiation detector 110 can move smoothly in at least one direction of the down direction M11 and the up direction M12 by tension applied by the spring balancer 163 according to the user's requirement.

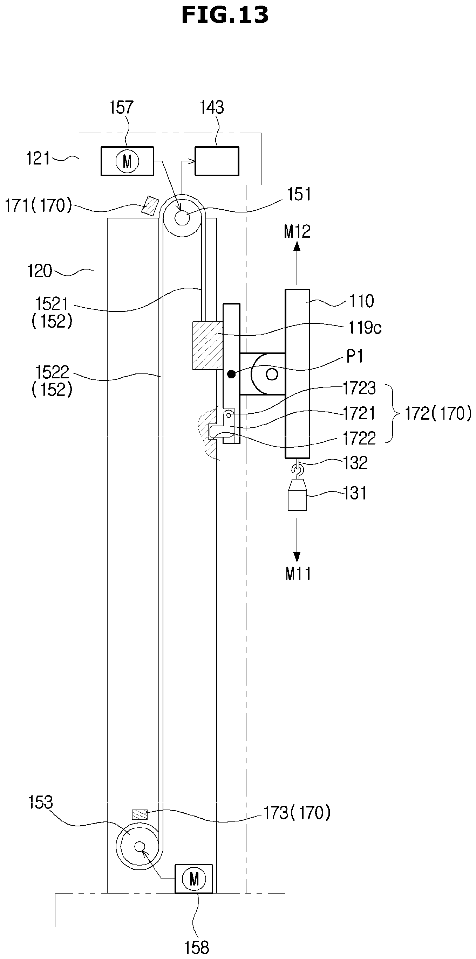

FIG. 13 is a view for describing a first support according to a fourth embodiment of the present disclosure.

As shown in FIG. 13, the first support 120 can include the detector moving member 150 to move the first weight adder 130 and the radiation detector 110 in at least one direction M1, and the first brake 170 to stop the radiation detector 110 at a specific position. According to an embodiment, the first support 120 can include the second weight adder 160, instead of the first weight adder 130.

The first weight adder 130 can apply a force to the radiation detector 110 in the direction M11 of gravity, and the second weight adder 160 can apply a force to the radiation detector 110 in the direction M12 that is opposite to the direction M11 of gravity.

The detector moving member 150 can include the one or more rotary bodies 151 and 153, and the moving body 152 coupled with the third coupling part 119c and configured to move the radiation detector 110 according to rotation of at least one of the rotary bodies 151 and 153.

The first brake 170 can include at least one of the first rotary body brake 171 disposed around the first rotary body 151, the side brake 172 disposed on one side of the first support housing 121, and the second rotary body brake 173 disposed around the second rotary body 153.

The first weight adder 130, the second weight adder 160, the detector moving member 150, and the first brake 170 have been described above, and accordingly, further descriptions thereof will be omitted.

According to an embodiment, the first support 120 can further include one or more motors 157 and 158 (that is, a first motor 157 and a second motor 158). The motors 157 and 158 can apply rotating power to the rotary bodies 151 and 152 to rotate the rotary bodies 151 and 152 in a predetermined direction, thereby moving the moving body 152 according to the rotation of the rotary bodies 151 and 152.

The motors 157 and 158 can provide rotation driving power to the corresponding rotary bodies (that is, the first rotary body 151 and the second rotary body 152), respectively. For example, the first motor 157 can provide driving power to the first rotary body 151, and the second motor 158 can provide driving power to the second rotary body 153. Any one of the first motor 157 and the second motor 158 can be omitted according to embodiments. The motors 157 and 158 can start operating according to control signals received from the driving controller 190 so as to transfer driving power to the rotary bodies 151 and 153. As such, the motors 157 and 158 can operate to move the radiation detector 110 in the up direction M12 or in the down direction M11.

If the radiation detector 110 is movable in the direction M11 of gravity by the weight of the radiation detector 110 and an additional weight, as shown in FIGS. 7 and 8, movement of the radiation detector 110 in the up direction M12, that is, in the direction M12 that is opposite to the direction M11 of gravity can be performed by the driving power of the motors 157 and 158. More specifically, if the motors 157 and 158 apply rotating power to the corresponding rotary bodies 151 and 153 in the predetermined directions r11 and r13, the one or more rotary bodies 151 and 153 can rotate in the predetermined directions r11 and r13 by the rotary power, and accordingly, the radiation detector 110 connected to the moving body 152 can move in the up direction M12. The predetermined directions r11 and r13 can be directions that are opposite to the rotation directions r12 and r14 of the rotary bodies 151 and 153 when the radiation detector 110 moves in the down direction M11.

If the radiation detector 110 is movable in the direction M12 that is opposite to the direction M11 of gravity, as shown in FIGS. 9 to 11, movement of the radiation detector 110 in the down direction M11 can also be performed by the driving power of the motor 157. More specifically, if the motor 157 provides rotating power to the rotary body 151 in the predetermined direction r12, the rotary body 151 can rotate in the predetermined direction r12 by the rotary power. The moving body 152 can move according to rotation of the rotary body 151, and the radiation detector 110 can move in the down direction M11. Herein, the predetermined direction r12 can be a direction that is opposite to the rotation direction r11 of the rotary body 151 when the radiation detector 110 moves in the up direction M12.

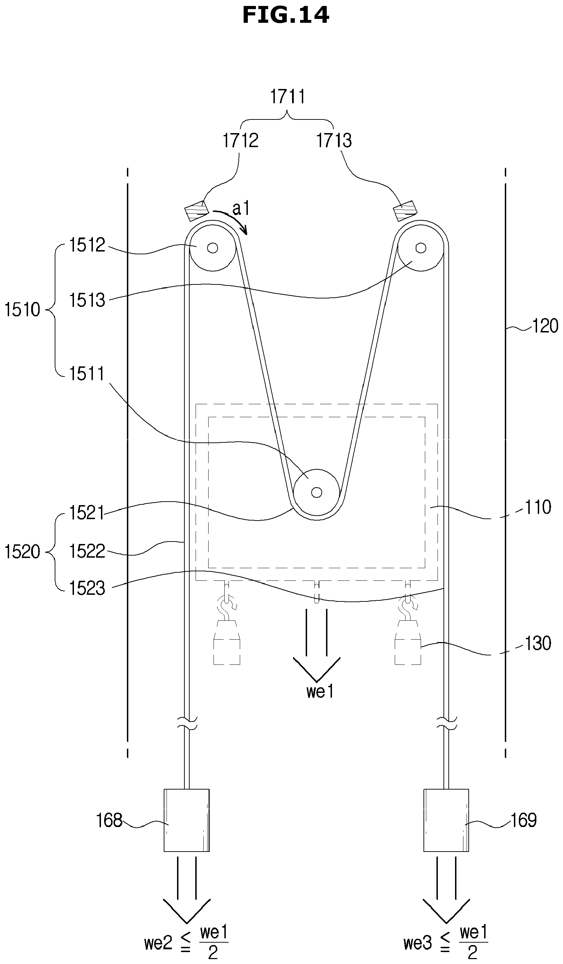

FIG. 14 shows a first rotary body according to another embodiment of the present disclosure.

As shown in FIG. 14, the detector moving member 150 of the radiation detection apparatus 100 can be implemented with a plurality of rotary bodies 1510, instead of the single rotary body 151. The plurality of rotary bodies 1510 can be disposed in the inside or outside of the first support 120. In this case, the detector moving member 150 can include a fourth rotary body 1511, a fifth rotary body 1512, and a sixth rotary body 1513.
The fourth rotary body 1511 can be implemented as a pulley, and disposed between the fifth rotary body 1512 and the sixth rotary body 1513. In this case, the fourth rotary body 1511 can be positioned at the middle portion of the moving body 152, and provided below the fifth rotary body 1512 and the sixth rotary body 1513, as shown in FIG. 12. According to movement of the moving body 152, the fourth rotary body 1511 can rotate to move in the up direction or in the down direction.

The fourth rotary body 1511 can be coupled with the third coupling part 119c connected to the radiation detector 110. Accordingly, the fourth rotary body 1511 can be interlocked with the radiation detector 110 to move in the up direction or in the down direction. A first weight We1 can be applied in the direction of gravity to the fourth rotary body 1511. The first weight We1 can be due to the weight of the radiation detector 110 and the weight of the moving body coupling member 119, or due to the weight of the radiation detector 110, the weight of the moving body coupling member 119, and the additional weight of the first weight adder 130 installed in the radiation detector 110.

The fifth rotary body 1512 and the sixth rotary body 1513 can be rotatably fixed at the first support 120. As described above, the fifth rotary body 1512 and the sixth rotary body 1513 can be disposed at a location that is relatively higher than that of the fourth rotary body 1511. The fifth rotary body 1512 and the sixth rotary body 1513 can be implemented as separate fixed pulleys. The moving body 152 can be wound on the fifth rotary body 1512 and the sixth rotary body 1513 such that the moving body 152 is movable in a predetermined direction according to rotation of the fifth rotary body 1512 and the sixth rotary body 1513. According to rotation of at least one of the fifth rotary body 1512 and the sixth rotary body 1513, the entire or a part of the moving body 152 can move, and accordingly, the fourth rotary body 1511 can also move in the up direction or in the down direction. For example, as shown in FIG. 14, if the fifth rotary body 1512 rotates in a counterclockwise direction a1, some parts 1521 and 1522 (also, referred to as first parts) of the moving body 152 can move in the rotation direction a1 of the fifth rotary body 1512, so that the fourth rotary body 1511 moves in the down direction.

The first part 1522 extending from one end of the moving body 152 can be wound on the fifth rotary body 1512 so that the first part 1522 is movable according to rotation of the fifth rotary body 1512. Tension corresponding to a second weight We2 can be applied in the direction of gravity to the first part 1522. According to an embodiment, the tension corresponding to the second weight We2 can be made by a weight 168 formed in the end of the first part 1522. Also, according to another embodiment, the end of the first part 1522 can be wound on the second rotary body 152, and the tension corresponding to the second weight We2 can be made by a friction force or a brake force of the second rotary body 153.

According to an embodiment, the second weight We2 can be equal to or smaller than half of the first weight We1 added to the fourth rotary body 1511.

A second part 1523 extending from the other end of the moving body 152 can be wound on the sixth rotary body 1513 so that the second part 1523 is movable according to rotation of the sixth rotary body 1513. Tension corresponding to a third weight We3 can be applied in the direction of gravity to the second part 1523. The tension corresponding to the third weight We3 can be made by a weight 169 formed in the end of the second part 1523. If the end of the second part 1523 is wound on the second rotary body 152, the tension corresponding to the third weight We3 can be made by a friction force or a brake force of the second rotary bod 152. In the embodiment in which the end of the first part 1522 and the end of the second part 1523 are wound on the second rotary body 152, a plurality of second rotary bodies 152 can be respectively provided in correspondence to the end of the first part 1522 and the end of the second part 1523 in the first support 120.

According to an embodiment, the third weight We3 can be equal to or smaller than half of the first weight We1 added to the fourth rotary body 1511. Also, the third weight We3 can be equal to or approximate the second weight We2. However, the second weight We2 can be different from the third weight We3, according to a designer's selection.

The radiographic detection apparatus 100 can include a rotary body brake 1711 to stop the fifth rotary body 1512 and the sixth rotary body 1513. The rotary body brake 1711 can include a fifth rotary body brake 1712 to stop the fifth rotary body 1512, and a sixth rotary body brake 1713 to stop the sixth rotary body 1513. The fifth rotary body brake 1712 and the sixth rotary body brake 1713 can respectively stop the fifth rotary body 1512 and the sixth rotary body 1513, independently or dependently.

Hereinafter, movement of the radiation detector 110 according to the above-described embodiment will be described under an assumption that the second weight We2 is equal to the first weight We1.

If the user moves the radiation detector 110 manually, both the fifth rotary body brake 1712 and the sixth rotary body brake 1713 can stop braking operation. In this case, the fifth rotary body brake 1712 and the sixth rotary body brake 1713 can stop braking operation, according to the control of the driving controller 190 and/or a controller 310 or according to a predetermined setting. Accordingly, the stopped states of the fifth rotary body 1512 and the sixth rotary body 1513 can be released. When the user moves the radiation detector 110 manually, neither the fifth rotary body brake 1712 nor the sixth rotary body brake 1713 can operate so that a relatively small weight can be added to the radiation detector 110. More specifically, a weight (We1-2*We2) corresponding to a difference between the first weight We1 and two times of the second weight We2, or a weight (We1-2*We3) corresponding to a difference between the first weight We1 and two times of the third weight We3 can be added to the radiation detector 110. Accordingly, the user can feel only the difference when moving the radiation detector 110 manually.

Meanwhile, when stitching scanning is performed, only any one of the fifth rotary body brake 1712 and the sixth rotary body brake 1713 can perform braking operation. In other words, only any one of the fifth rotary body 1512 and the sixth rotary body 1513 can be set to rotate. If the fifth rotary body 1512 is stopped and the sixth rotary body 1513 is rotatable, a weight ({We1-2*We3}/2) corresponding to half of the difference between the first weight We1 and two times of the third weight We3 can be added to the radiation detector 110. In contrast, if the fifth rotary body 1512 is rotatable and the sixth rotary body 1513 is stopped, a weight ({We1-2*We2}/2) corresponding to half of the difference between the first weight We1 and two times of the second weight We2 can be added to the radiation detector 110. Accordingly, the radiation detector 110 can be movable in the down direction according to the added weight.

In this case, according to an embodiment, the fifth rotary body 1512 and the sixth rotary body 1513 can be stopped alternately. In other words, if a predetermined time period elapses after the fifth rotary body 1512 starts rotating, or if the fifth rotary body 1512 rotates more than a predetermined number of revolution, the fifth rotary body 1512 can stop rotating, and the sixth rotary body 1513 can be released from its stopped state to start rotating. If a predetermined time period elapses after the sixth rotary body 1513 starts rotating, or if the sixth rotary body 1513 rotates more than a predetermined number of revolution, the sixth rotary body 1513 can be again stopped, and the fifth rotary body 1512 can be released from its stopped state to again start rotating.

According to the above-described method, the position of the radiation detector 110 can easily change manually or automatically, and accordingly, the user's convenience in manipulating the radiation detection apparatus 100 can be improved.

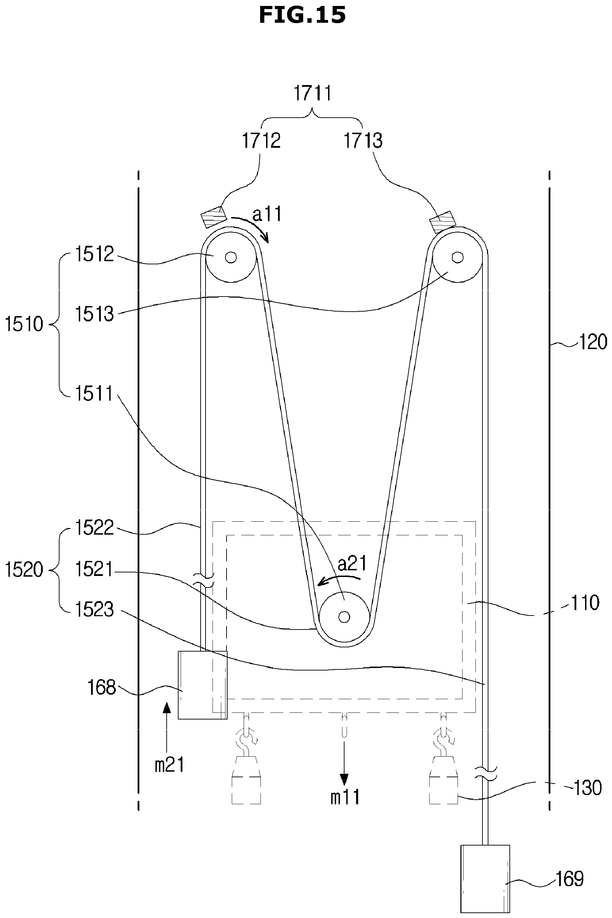

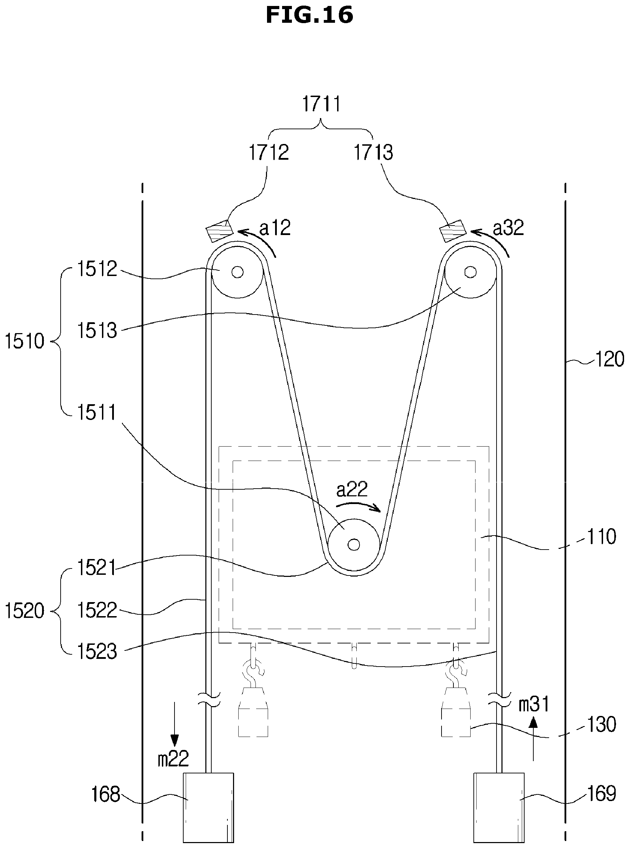

FIG. 15 is a first view for describing an example of driving of a first rotary body according to another embodiment of the present disclosure, and FIG. 16 is a second view for describing an example of driving of the first rotary body according to the other embodiment of the present disclosure.

As shown in FIGS. 15 and 16, if the detector moving member 150 is implemented with a plurality of rotary bodies 1510, the fifth rotary body brake 1712 and the sixth rotary body brake 1713 can be sequentially released from their braking operations.

As shown in FIG. 15, if the fifth rotary body brake 1712 is released, the fifth rotary body 1512 can become rotatable. In this case, the sixth rotary body brake 1713 can still maintain its braking operation so that the sixth rotary body 1513 is maintained at its stopped state. If the fifth rotary body 1512 becomes rotatable, no supporting force can be applied to the radiation detector 110 so that the radiation detector 110 moves in the down direction M11 by the weight of the radiation detector 110 and an additional weight added by the first weight adder 130. The fourth rotary body 1511 can rotate in a predetermined direction a21 according to the movement of the radiation detector 110, and the fifth rotary body 1512 can also rotate in a predetermined direction a11 according to movement of the moving body 152 interworking with the movement of the radiation detector 110. The weight 168 formed in the end of the first part 1522 can move in the up direction m21 according to the movement of the radiation detector 110 and the rotation of the fifth rotary body 1512.

If the radiation detector 110 moves by a predetermined distance, the radiation detector 110 can be stopped by the first brake 170, for example, by the side brake 172.

If the radiation detector 110 arrives at a predetermined position and stops, the sixth rotary body brake 1712 can also stop braking, and accordingly, the sixth rotary body 1513 can also become rotatable. Accordingly, all of the fourth to sixth rotary bodies 1511 to 1513 can become rotatable, and the individual weights 168 and 169 can move such that the weight of the weight 168 formed in the end of the first part 1522, the weight of the radiation detector 110, the additional weight added by the first weight adder 130, and the weight of the weight 169 formed in the end of the second part 1523 can be balanced. For example, the weight 168 formed in the end of the first part 1522 can move in the down direction m22, and the weight 169 formed in the end of the second part 1523 can move in the up direction m31. The individual rotary bodies 1511, 1512, and 1513 can rotate in correspondence to the movement of the weights 168 and 169. In this case, the radiation detector 110 can little move by the braking operation of the first brake 170, for example, the side brake 172. If the radiation detector 110 is balanced with the weights 168 and 169 by the movement of the weights 168 and 169, the fifth rotary body brake 1712 and the sixth rotary body brake 1713 can start braking operation, and accordingly, the radiation detector 110 can be stopped and disposed at a predetermined position stably.

The embodiments shown in FIGS. 14 to 16 can be applied in the same manner to the radiation irradiation apparatus 200, as well as the radiation detection apparatus 100. The above-described embodiments can be applied to a second support 220 of the radiation irradiation apparatus 200 in the same manner or through slight modification, so that the location of the radiation emitter 210 can be easily changed manually or automatically.

According to an embodiment, the radiation detection apparatus 100 can further include a position detector 140 to detect a moved position of the radiation detector 110.

The position detector 140 can detect a position of the radiation detector 110 moving or stopped, output an electrical signal corresponding to the result of the detection, and transfer the electrical signal to the driving controller 190 and/or the controller 310. The driving controller 190 and/or the controller 310 can decide a position of the radiation detector 110 based on the received electrical signal, and transfer a control signal related to operation of the first brake 170 to the first brake 170 according to the decided position of the radiation detector 110.

According to an embodiment, the position detector 140 can directly transfer an electrical signal related to the result of the detection to the first brake 170. In this case, the first brake 170 can operate according to a predetermined setting based on the received electrical signal to stop the radiation detector 110 at a predetermined position.

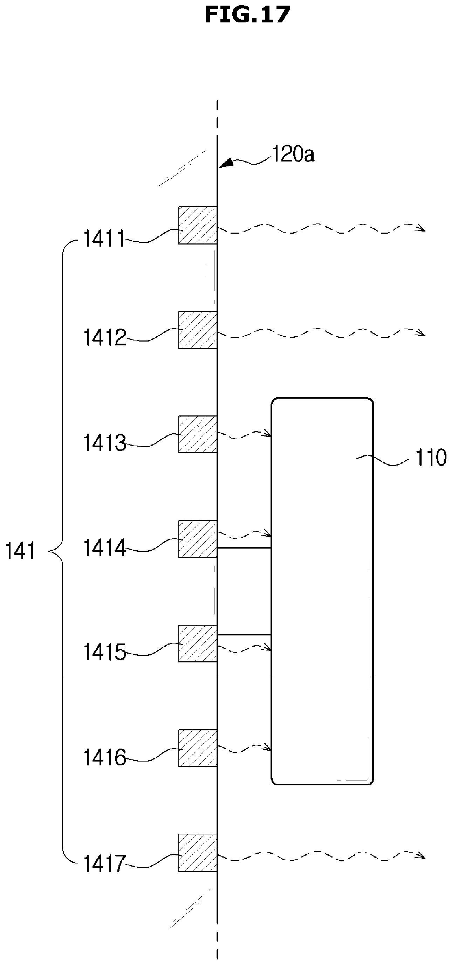

According to an embodiment, the position detector 140 can be, as shown in FIGS. 17, 18, and 19, implemented as at least one of a moved position detector 141, a moved distance detector 142, and a rotation angle detector 143.

FIG. 17 is a view for describing a method of detecting a position of a radiation detector, according to a first embodiment of the present disclosure.

The moved position detector 141 can detect a moved position of the radiation detector 110. The moved position detector 141 can detect a position of the radiation detector 110 using a visible light sensor, an infrared light sensor, or an ultrasound sensor.

The moved position detector 141 can be, as shown in FIG. 17, implemented using a plurality of sensors 1411 to 1417 arranged on one surface of the first support housing 121. The surface of the first support housing 121 can include the surface of the first support housing 121 on which the radiation detector 110 is located. The individual sensors 1411 to 1417 can emit visible light, infrared light, or ultrasonic waves, receive the visible light, infrared light, or ultrasonic waves reflected from the rear surface of the radiation detector 110, generate an electrical signal corresponding to the received visible light, infrared light, or ultrasonic waves, and then output the electrical signal. In this case, the electrical signal can further include an identification number for identifying the sensors 1411 to 1417 outputting the electrical signal.

The driving controller 190 can acquire a position of the radiation detector 110 based on the electrical signal, and control the radiation detector 110 to stop based on the acquired position of the radiation detector 110. For example, the driving controller 190 can identify a sensor(s) (for example, the third to sixth sensors 1413 to 1416) outputting the electrical signal, from among the plurality of sensors 1411 to 1417, and detect a current position of the radiation detector 110 based on the absolute or relative positions of the identified sensors 1413 to 1416.

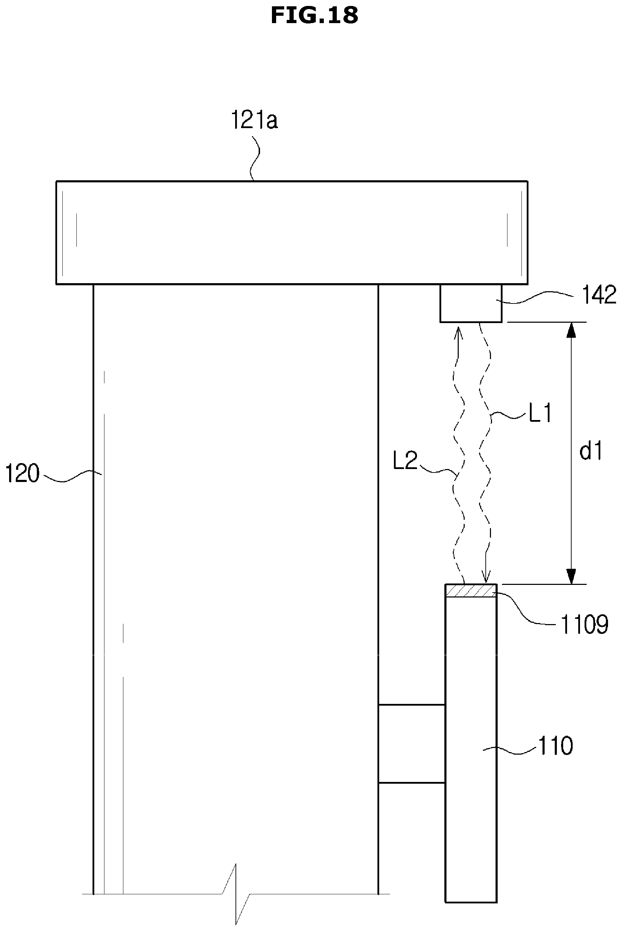

FIG. 18 is a view for describing a method of detecting a position of a radiation detector, according to a second embodiment of the present disclosure.

The moved distance detector 142 can sense a moved distance of the radiation detector 110, and calculate a position of the radiation detector 110. The moved distance detector 142 can detect a position of the radiation detector 110 using, for example, a visible light sensor, an infrared light sensor, or an ultrasound sensor.

The moved distance detector 142 can be, as shown in FIG. 18, installed in the top 121 a of the first support 120 so that the moved distance detector 142 can emit light or ultrasonic waves toward the radiation detector 110 on the moving line of the radiation detector 110.

The moved distance detector 142 can emit visible light, infrared light, or ultrasonic waves L1 toward the radiation detector 110, and then receive visible light, infrared light, or ultrasonic waves L2 reflected from the radiation detector 110. When the visible light, infrared light, or ultrasonic waves L1 are emitted, the radiation detector 110 can acquire information about a time at which the visible light, infrared light, or ultrasonic waves L1 are emitted, and when the visible light, infrared light, or ultrasonic waves L2 are received, the radiation detector 110 can acquire information about a time at which the visible light, infrared light, or ultrasonic waves L2 are received, and then transfer the acquired information to the driving controller 190. The driving controller 190 can calculate a distance between the top of the radiation detector 110 and the moved distance detector 142, based on the information about the time at which the visible light, the infrared light, or the ultrasonic waves L1 are emitted and the information about the time at which the visible light, infrared light, or ultrasonic waves L2 are received to calculate a moved distance d1 of the radiation detector 110, and acquire an absolute or relative position of the radiation detector 110 based on the calculated moved distance d1.

According to an embodiment, on the top surface of the radiation detector 110, a reflective surface 1109 can be further formed to easily reflect visible light, infrared light, or ultrasonic waves emitted from the moved distance detector 142.

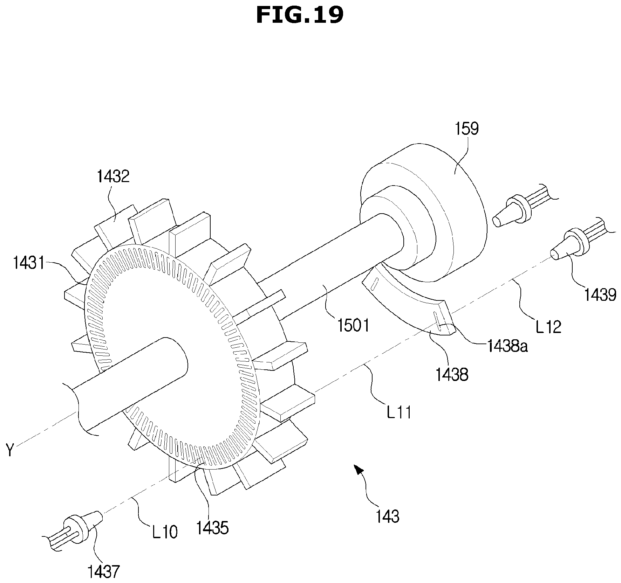

FIG. 19 is a view for describing a method of detecting a position of a radiation detector, according to a third embodiment of the present disclosure.

The rotation angle detector 143 can be configured to sense the number of revolution or the amount of rotation (hereinafter, referred to as the number of revolution) of at least one rotary body (for example, at least one of the first rotary body 151 and the second rotary body 153) installed in the radiation detection apparatus 100. The rotation angle detector 143 can be installed around the rotary bodies 151 and 153 for which the number of revolution is to be sensed, and sense what times the rotary bodies 151 and 153 rotate or what angle the rotary bodies 151 and 153 rotate to.

According to an embodiment, the rotation angle detector 143 can be implemented as a rotary encoder. The rotary encoder can be implemented as an optical incremental encoder, an optical absolute encoder, a magnetic encoder, and/or a resolver.

If the rotation angle detector 143 is implemented as an optical encoder, the rotation angle detector 143 can include, for example, a rotary body 1431, at least one first slot 1435 formed in the rotary body 1431 in such a way to penetrate the rotary body 1431, at least one light source 1437 to irradiate light L10 toward the first slots 1435, a fixing slot member 1438 in which at least one second slot 1438a is formed to pass light L11 irradiated from the at least one light source 1437 and then passed through the first slots 1435, and at least one light detector 1439 to detect light L12 passed through the second slot 1438a of the fixing slot member 1438.

If the rotary body 1431 rotates, the first slot 1435 can also rotate accordingly. If the first slot 1435 is aligned to the light source 1437, the second slot 1438a, and the light detector 1439, the light L10 irradiated from the light source 1437 can pass through the second slot 1438a and be transferred to the light detector 1439. Accordingly, the light detector 1439 can detect light L12. In contrast, if the first slot 1435 is not aligned to the light source 1437, the second slot 1438a, and the light detector 1439, the light L11 passed through the first slot 1435 cannot pass through the second slots 1438a, and accordingly, the light detector 1439 cannot detect any light.

The light detector 1439 can output an electrical signal corresponding to the detected light in the form of a pulse. The number of revolution of the rotary body 1431 can be measured by counting the number of pulses output from the light detector 1439, and the speed of revolution of the rotary body 1431 can also be measured in the same way. The electrical signal can be transferred to the driving controller 190 and/or the controller 310, and the driving controller 190 and/or the controller 310 can measure a moved distance of the moving body 152 based on the number of revolution or the speed of revolution of the rotary body 1431. Since the moved distance of the moving body 152 corresponds to a moved distance of the radiation detector 110, the driving controller 190 and/or the controller 310 can calculate a relative or absolute position of the radiation detector 110 using the moved distance of the moving body 152.

If a plurality of light sources 1437, a plurality of second slots 1438a, and a plurality of light detectors 1439 are provided, the respective light detectors 1439 can output electrical signals of a plurality of channels having different phases in the form of pulses, according to rotation of the rotary body 1431. The electrical signals of the plurality of channels can be used to measure the rotation direction of the rotary body 1431. The driving controller 190 and/or the controller 310 can further use the rotation direction of the rotary body 1431 to calculate a relative or absolute position of the radiation detector 110.

Also, the position detector 140 can be implemented as various means capable of detecting a position, in addition to the above-described embodiments. For example, the position detector 140 can be implemented as a potentiometer (POT). The potentiometer is a sensor capable of outputting an electrical signal of a voltage corresponding to mechanical rotational displacement. Since a voltage of an electrical signal output from the potentiometer is proportional to rotational displacement, the driving controller 190 and/or the controller 310 can measure an amount of revolution of the rotary bodies 151 and 153 based on the electrical signal output from the potentiometer, and acquire a moved distance of the radiation detector 110 based on the measured amount of revolution.

Accordingly, a relative or absolute position of the radiation detector 110 can be calculated. Also, any other means capable of sensing the position of the radiation detector 110 can be used as the position detector 140 of the radiation detection apparatus 100.

Also, the radiation detection apparatus 100 can further include the driving controller 190 to control movement of the radiation detector 110, as necessary.

The driving controller 190 can process various information related to operations of the radiation detection apparatus 100, and also control overall operations of the radiation detection apparatus 100.

For example, the driving controller 190 can transfer a control signal to the first brake 170 to control the first brake 170, so that the radiation detector 110 can be stopped at one or more positions, such as at least one position.

According to an embodiment, the driving controller 190 can receive an electrical signal corresponding to the result of detection by the position detector 140, and generate a control signal (that is, a brake signal) for operating the first brake 170, based on the received electrical signal. In this case, the driving controller 190 can decide a position of the radiation detector 110 according to the result of detection by the position detector 140, and generate a brake signal for the first brake 170 according to the result of the decision. More specifically, the driving controller 190 can compare the decided position to a predetermined scanning position, and if the driving controller 190 determines that the decided position is identical to the predetermined scanning position, the driving controller 190 can generate a brake signal for the first brake 170.

The brake signal can be transferred to the first brake 170, and the first brake 170 can operate according to the brake signal to stop the radiation detector 110. Accordingly, the radiation detector 110 can stop at a specific position, for example, at a scanning position when moving in the down direction M11 by an additional weight provided by the first weight adder 130, or when moving in the up direction M12 by an additional weight provided by the second weight adder 160

According to another example, the driving controller 190 can generate a control signal for the motors 157 and 158 of the detector moving member 150, according to a control signal transferred from the control apparatus 300 or according to a predetermined setting or a user's instruction, and transfer the control signal to the motors 157 and 158. Accordingly, the radiation detector 110 can move in the up direction M12 even when an additional weight is added by the first weight adder 130, and also can move in the down direction M11 even when an additional weight is added by the second weight adder 160.

Also, according to another embodiment, the driving controller 190 can control the radiation detector 110 to stop using a clock (not shown). For example, if the radiation detector 110 starts moving, the driving controller 190 can recognize that the radiation detector 110 starts moving, and record information about a time at which the radiation detector 110 starts moving, based on information about a time received from the clock. If a reference time period elapses after the radiation detector 110 starts moving, the driving controller 190 controls the first brake 170 to stop the radiation detector 110. If the moving speed of the radiation detector 110 has already been known, a moved distance of the radiation detector 110 according to elapse of time can also be calculated, and a current relative or absolute position of the radiation detector 110 can be calculated based on the moved distance of the radiation detector 110. Accordingly, the driving controller 190 enables the radiation detector 110 to stop at a desired position. The reference time may have been calculated in advance using the moving speed of the radiation detector 110 and a desired moving distance. The moving speed of the radiation detector 110 can have been decided theoretically or experientially.

According to an embodiment, the driving controller 190 controls the radiation detector 110 to be located at a position corresponding to the position of the radiation emitter 210 and/or a degree of tilting of the radiation emitter 210. For example, the driving controller 190 can receive information about the position of the radiation emitter 210 or about a degree of tilting of the radiation emitter 210 from the radiation irradiation apparatus 200 or the controller 310, decide a position of the radiation detector 110 based on the received information, and control the first brake 170 to stop the radiation detector 110 at the decided position.

In this case, if the radiation detector 110 moves in the down direction M11 by the weight of the radiation detector 110 and/or the additional weight provided by the first weight adder 130, or moves in the up direction M12 by the additional weight provided by the second weight adder 160, the driving controller 190 transfers a control signal to the first brake 170 to locate the radiation detector 110 at a position corresponding to the position of the radiation emitter 210 and/or the degree of tilting of the radiation emitter 210. According to an embodiment, the driving controller 190 transfers a control signal to the motors 157 and 158 to control the motors 157 and 158 to move the radiation detector 110 to a position corresponding to the position of the radiation emitter 210 and/or a degree of tilting of the radiation emitter 210.

The driving controller 190 can be implemented with a processor composed of at least one semiconductor chip and the related components. The processor can be configured to execute one or more programs according to a predetermined procedure.

According to another embodiment, the driving controller 190 may be omitted, and in this case, a driving controller 290 of the radiation irradiation apparatus 200 and/or the controller 310 of the control apparatus 300 perform the above-described operations of the driving controller 190.

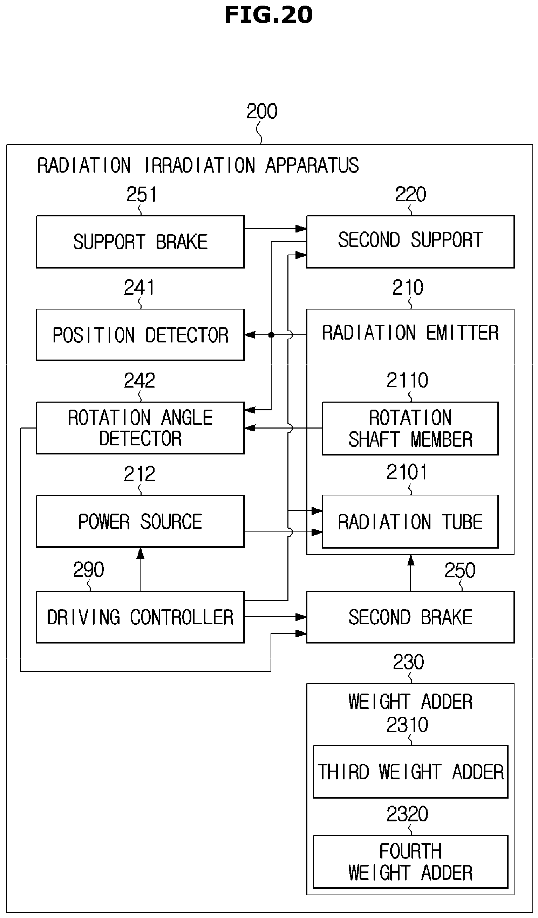

Hereinafter, the radiation irradiation apparatus 200 will be described in detail with respect to FIG. 20.

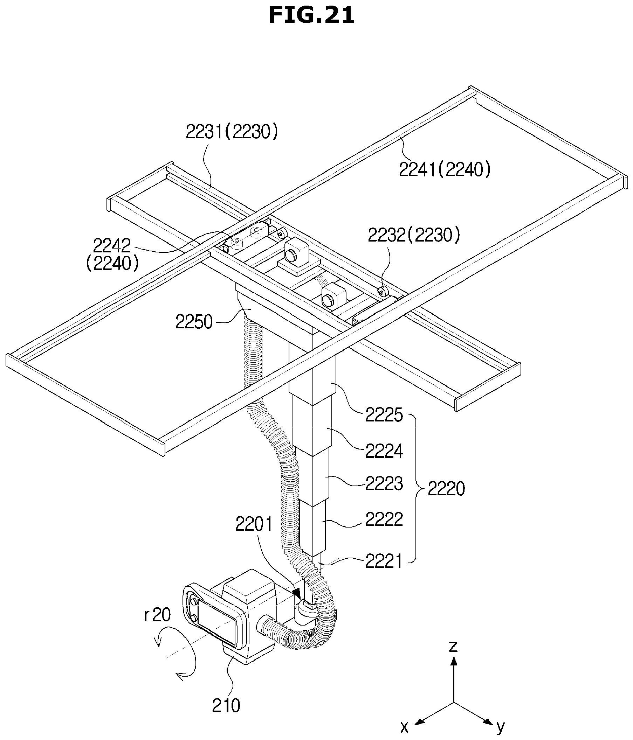

FIG. 20 is a control block diagram of a radiation emitter according to an embodiment of the present disclosure, and FIG. 21 is a perspective view of a radiation emitter according to an embodiment of the present disclosure.

As shown in FIGS. 20 and 21, the radiation irradiation apparatus 200 can irradiate radiation toward a subject. The radiation irradiation apparatus 200 can include the radiation emitter 210 to emit radiation, and the second support 220 in which the radiation emitter 210 is installed. Also, the radiation irradiation apparatus 200 can further include one or more support moving parts 2230 and 2240 to change a position of the second support 220 on an x-y plane, and a weight adder 230 to apply an additional weight to at least one of the radiation emitter 210 and the second support 220, as necessary.

Also, the radiation irradiation apparatus 200 can further include at least one of a second brake 250 to stop the rotational movement of the radiation emitter 210, and a support brake 251 to stop extending and/or shortening the length of the second support 220.

Also, the radiation irradiation apparatus 200 can further include at least one of a position detector 241 to detect a position of the radiation emitter 210, and a rotation angle detector 242 to detect a rotation angle of the radiation emitter 210.

Also, the radiation irradiation apparatus 200 can further include at least one of a power source 212 to supply power to a radiation tube 2101, and the driving controller 290 to control movement of the radiation emitter 210, as necessary.

The radiation emitter 210 can include the radiation tube 2101 to generate radiation according to a voltage and/or current applied from the power source 212, and to emit the radiation. The radiation tube 2101 can emit radiation of energy corresponding to a tube voltage and/or tube current according to the magnitude of an applied voltage and/or current. The radiation generated by the radiation tube 2101 can be emitted to the outside through the irradiation surface of the radiation emitter 210, and irradiated toward a subject. The irradiation range and irradiation direction of the radiation emitted from the radiation tube 2101 can be adjusted by a collimator (not shown), etc.

The radiation emitter 210 can be connected to the second support 220. For example, the radiation emitter 210 can be connected to one end 2201 of the second support 220. According to another embodiment, the radiation emitter 210 is connected to the middle portion of the second support 220.

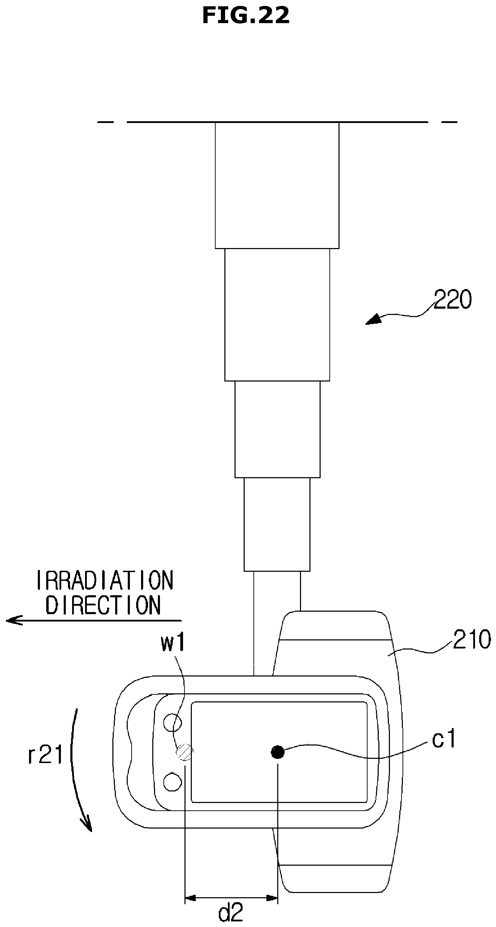

FIG. 22 is a view for describing an example of rotation of a radiation emitter.

Referring to FIG. 22, the radiation emitter 210 can rotate in a predetermined direction r21 with respect to a predetermined axis c1 of rotation. In order to rotate the radiation emitter 210 in the predetermined direction r21 with respect to the predetermined axis c1 of rotation, the outer housing of the radiation emitter 210 can be coupled with a shaft member (not shown). One end of the shaft member can be coupled with the outer housing of the radiation emitter 210, and the other end of the shaft member can be rotatably connected to an emitter coupling housing 2210 formed in the middle portion or end of the second support 220. The radiation emitter 210 can rotate with respect to the axis c1 of rotation, and then stop to irradiate radiation at a predetermined irradiation angle. The irradiation angle means an angle formed between the irradiation direction of the radiation emitter 210 and the ground. The irradiation angle of the radiation emitter 210 can be decided according to the position of the radiation detector 110. According to an embodiment, the irradiation angle of the radiation emitter 210 can be decided by the driving controller 290 and/or the controller 310. At least one of the position of the radiation detector 110 210 and the irradiation angle of the radiation emitter 210 can have been defined in advance according to a user's or designer's selection.

According to an embodiment, in the other end of the shaft member, a motor for rotating the radiation emitter 210 can be further provided. The motor can be installed in the emitter coupling housing 2210. The motor can operate to apply rotating power to the shaft member, and the shaft member can rotate by the rotating power applied by the motor. The radiation emitter 210 coupled with the shaft member can also rotate in the predetermined direction r21 in correspondence to the rotation of the shaft member.

The center w1 of gravity of the radiation emitter 210 can exist at a point spaced by a predetermined distance d2 from the axis c1 of rotation of the radiation emitter 210. In this case, the center w1 of gravity can be leaning in the irradiation direction of radiation with respect to the axis c1 of rotation. Since the center w1 of gravity is spaced by the predetermined distance d2 from the axis c1 of rotation, the radiation emitter 210 can rotate in a predetermined direction r21 by the force of gravity unless the second brake 150 is released and the motor does not operate. In this case, the radiation emitter 210 can be tilted with respect to the axis c1 of rotation, so that one end of the radiation emitter 210 located in the irradiation direction of radiation, that is, the end of the radiation emitter 210 on which the irradiation surface is formed can face the down direction, and the other end of the radiation emitter 210 can face the up direction. In this case, if a friction force between the shaft member and a surface which is provided in the emitter coupling housing 2210 and on which the shaft member is located is sufficiently great, the radiation emitter 210 can not rotate in spite of the distance d2 between the center w1 of gravity and the axis c1 of rotation.

The second support 220 can be connected to the radiation emitter 210, and change the position of the radiation emitter 210 or the irradiation direction of the radiation emitter 210. In the middle portion or end of the second support 220, the emitter coupling housing 2210 in which the shaft member having one end connected to the radiation emitter 210 is inserted can be provided.

The second support 220 can include a post frame 2220 that can extend or be shortened, a moving carriage 2250 coupled with the top of the post frame 2220, and one or more support moving parts 2230 and 2240 to move the post frame 220 in a predetermined direction on the x-y plane.

The post frame 2220 can extend by the weight of the radiation emitter 210, or by the weight of the radiation emitter 210 and an additional weight applied by a fourth weight adder 2320 (see FIG. 20).

The post frame 2220 can include a plurality of posts 2221 to 2225. The posts 2221 to 2224 except for the uppermost post 2225 can be inserted into or drawn from any one of the other posts 2222 to 2225. In contrast, the other posts 2222 to 2225 except for the lowermost post 2221 can be inserted into or drawn from any one of the other posts 2222 to 2225. When the posts 2211 to 2225 are inserted or drawn, the length of the post frame 2220 can extend or be shortened.

The radiation emitter 210 can be connected to one end 2201 of the post frame 2220, and the other end 2202 of the post frame 220 can be connected to the moving carriage 2250. The moving carriage 2250 can be coupled with rails 2231 and 2241 in such a way to be movable along the rails 2231 and 2241.

In the inside of the moving carriage 2250, at least one rotary body (not shown) can be disposed, wherein the at least one rotary body can be a pulley, a toothed wheel, or the like. The pulley or the toothed wheel can be wound with an appropriate cable, wire, belt, chain, or the like. The cable can move according to rotation of the pulley or the toothed wheel. At least one end of the cable can extend to the inside of the post frame 2220 and be coupled with one end 2201 of the post frame 2220. If the pulley or the toothed wheel rotates in a predetermined direction, the end 2201 of the post frame 2220 can move in the up direction or in the down direction in correspondence to the rotation direction of the pulley or the toothed wheel, so that the radiation emitter 210 can also move in the up direction or in the down direction according to the movement of the end 2201 of the post frame 2210. The radiation emitter 210 can move in the down direction by the additional weight applied by the fourth weight adder 232, and the cable can also move in correspondence to the movement of the radiation emitter 210. In this case, the pulley or the toothed wheel can rotate according to the movement of the cable.

On the top of the moving carriage 2250, one or more support moving parts 2230 and 2240 can be disposed. The support moving parts 2230 and 2240 can move the moving carriage 2250 to a predetermined position on the x-y plane so that the radiation emitter 210 is disposed at a predetermined position on the x-y plane. The support moving parts 2230 and 2240 can include one or more rails 2231 and 2241, and one or more driving bodies 2232 and 2242 traveling along the rails 2231 and 2241. A part of the driving bodies 2232 and 2242 can be formed on the top surface of the moving carriage 2250. The rails 2231 and 2241 can be disposed or positioned at right angles to each other, as shown in FIG. 21. The driving bodies 2232 and 2242 can include a wheel moving on the rails 2231 and 2241, and a motor to rotate the wheel.

The weight adder 230 can be provided to apply an additional weight to the radiation emitter 210, and can apply a weight to the second support 220, as necessary.

As shown in FIG. 20, the weight adder 230 can include at least one of a third weight adder 2310 and a fourth weight adder 2320.

The third weight adder 2310 can add an additional weight to the radiation emitter 210 so as to apply great rotating power to the radiation emitter 210. The radiation emitter 210 can acquire great rotating power exceeding the friction force between the shaft member and the surface on which the shaft member is located, by the third weight adder 2310, and accordingly, the radiation emitter 210 can rotate in the predetermined direction r21.

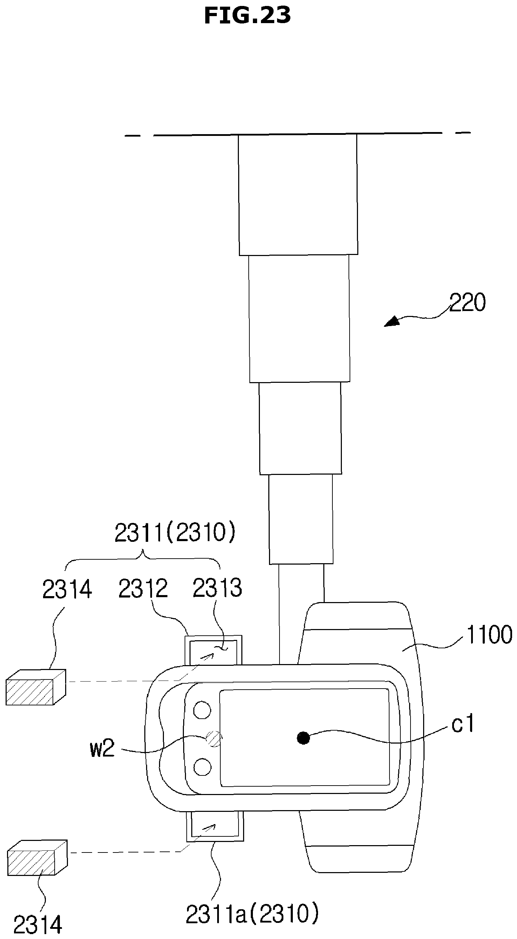

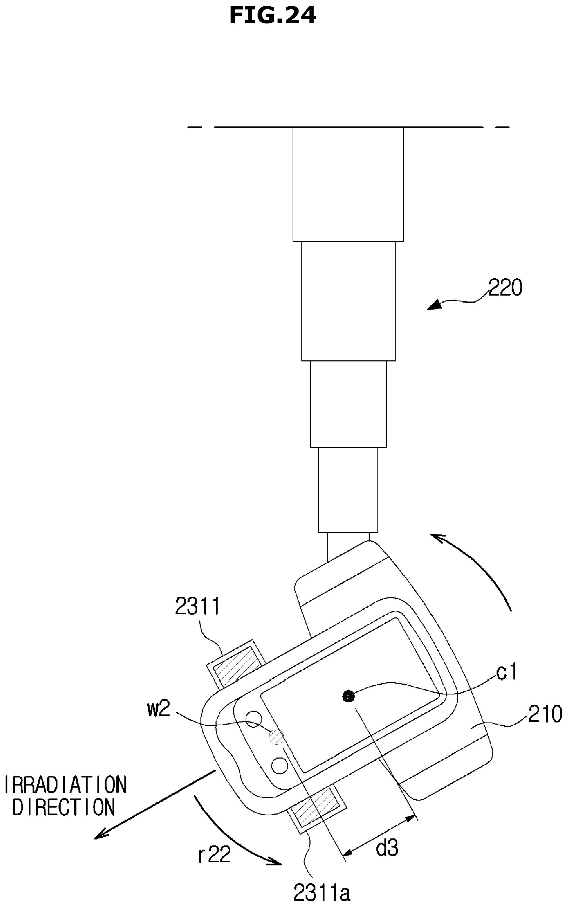

FIG. 23 shows a third weight adder installed in a radiation emitter, according to a first embodiment of the present disclosure, and FIG. 24 is a view for describing an example of rotation of the radiation emitter in which the third weight adder according to the first embodiment of the present disclosure is installed.

Referring to FIGS. 23 and 24, a third weight adder 2310 according to a first embodiment of the present disclosure can further include a weight 2314, and a weight installing member 2312 provided in the outer housing of the radiation emitter 210. The weight installing member 2312 can be formed in the radiation emitter 210, adjacent to the radiation irradiation surface with respect to the axis c1 of rotation. In the weight installing member 2312, resting space can be formed in which the weight 2314 can be inserted and rested. According to an embodiment, the weight installing member 2312 can further include a cover to open or close the resting space.

If the weight 2314 is inserted in the weight installing member 2312, the weight 2314 can apply a greater force in the direction of gravity to one end of the radiation emitter 210 on which the radiation irradiation surface is formed. Accordingly, the center w2 of gravity of the radiation emitter 210 can be located farther from the axis c1 of rotation than when no weight 2314 is installed (that is, d3>d2). Accordingly, greater rotating power can be applied to the radiation emitter 210, and if no motor operates or if the second brake 250 does not operate, the radiation emitter 210 can rotate smoothly in a predetermined direction r22 with respect to the axis c1 of rotation, as shown in FIG. 24. In this case, a direction in which the radiation irradiation surface faces, that is, a direction in which radiation is irradiated can change gradually to the down direction, that is, a direction facing the ground.

According to an embodiment, the third weight adder 2310 can be configured to install the weight 2314 in the outer housing of the radiation emitter 210 using magnetism, or to install the weight 2314 in the outer housing of the radiation emitter 210 by various methods that can be consider by a designer.

A single weight adder 2310 or a plurality of weight adders 2310 can be installed in the radiation emitter 210, according to embodiments.

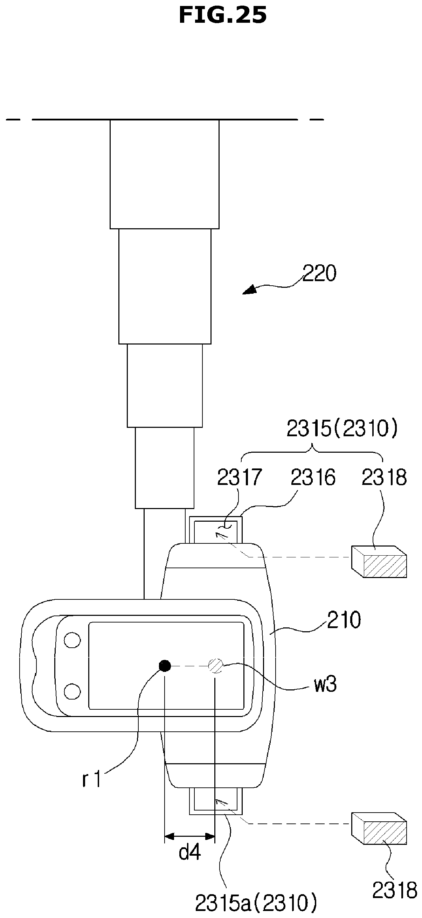

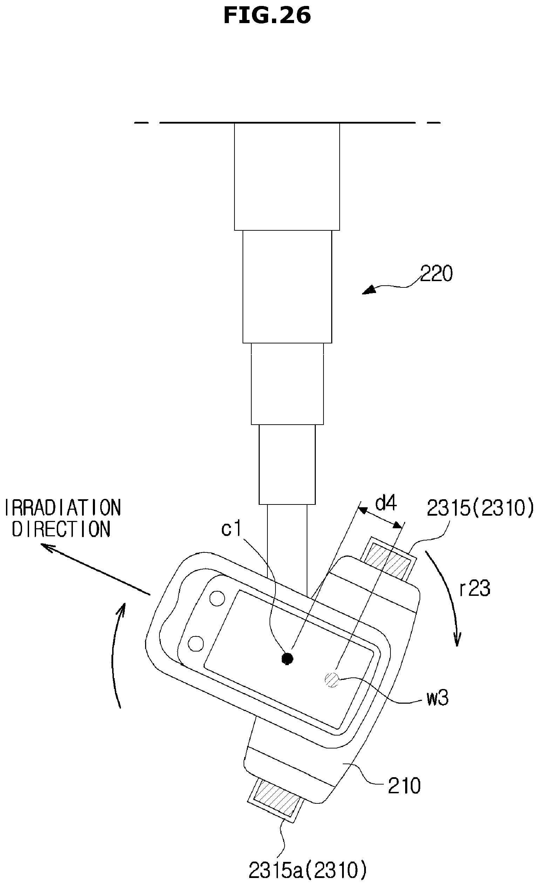

FIG. 25 shows a second embodiment of a weight adder installed in a radiation emitter, and FIG. 26 is a view for describing an example of rotation of a radiation emitter to which the weight adder according to the second embodiment of the present disclosure is added.

Referring to FIGS. 25 and 26, one or more third weight adders 2315 and 2315a according to a second embodiment of the present disclosure can further include a weight installing member 2317 installed in the outer housing of the radiation emitter 210, and a weight 2318 installed in the weight installing member 2317 and adding an additional weight to the radiation emitter 210. The weight installing member 2317 can be installed in the radiation emitter 210 at a location adjacent to the opposite end of the radiation irradiation surface of the radiation emitter 210. The weight installing member 2317 can include resting space 2316 in which the weight 2318 can be inserted and rested, and further include a cover to open or close the resting space 2316, as necessary.

If the weight 2318 is inserted in the weight installing member 2317, the weight 2318 can apply a force to the opposite end of the radiation irradiation surface of the radiation emitter 210, in the direction of the ground. Accordingly, the center w3 of gravity of the radiation emitter 210 can exist in a direction that is opposite to the direction of the center w3 of gravity of the radiation emitter 210 when no weight 2314 is installed or when the third weight adder 2310 according to the first embodiment is installed. In this case, a distance d4 between the center w3 of gravity and the axis c1 of rotation can be shorter or longer than when no weight 2314 is installed or when the third weight adder 2310 according to the first embodiment is installed. As such, by applying an additional weight to the opposite end of the radiation irradiation surface, rotating power can be applied to the radiation emitter 210 in a direction that is opposite to the direction in which rotating power is applied to the radiation emitter 210 when no weight 2314 is installed or when the third weight adder 2310 according to the first embodiment is installed. If no motor operates, or if the second brake 250 does not operate, the radiation emitter 210 can rotate in a direction r23, as shown in FIG. 26. In this case, the direction of the radiation irradiation surface, that is, the direction in which radiation is irradiated can change gradually to face a direction that is opposite to the direction of the ground.

A single weight adder 2315 and 2315a according to the second embodiment of the present disclosure can be installed in the radiation emitter 210, or two or more weight adders 2315 and 2315a according to the second embodiment of the present disclosure can be installed in the radiation emitter 210.

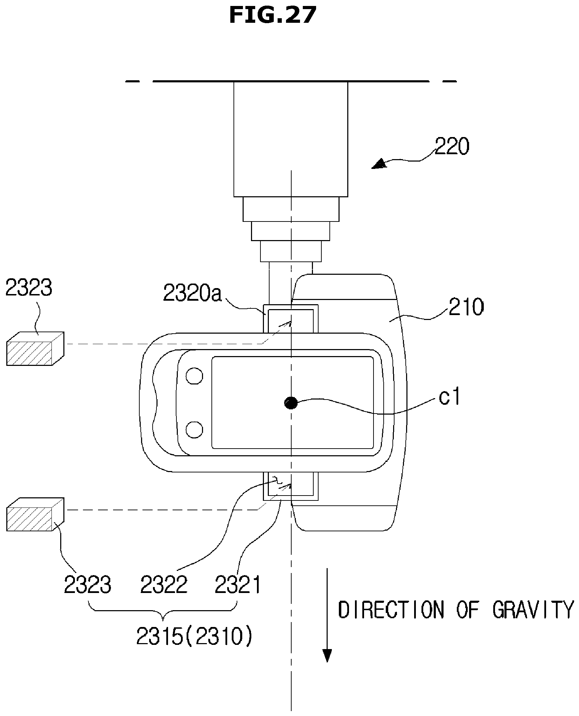

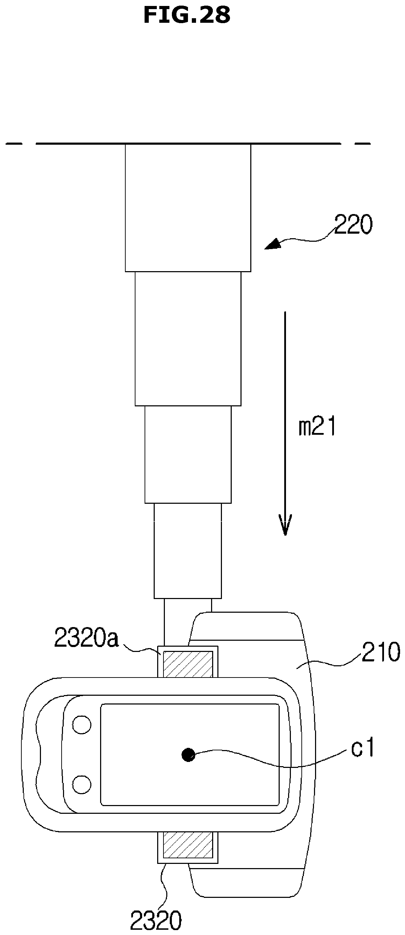

FIG. 27 shows an embodiment of a fourth weight adder installed in a radiation emitter, and FIG. 28 is a view for describing an example in which a second support extends.

Referring to FIGS. 27 and 28, one or more fourth weight adders 2320 and 2320a can add an additional weight to the radiation emitter 210. The fourth weight adders 2320 and 2320a can be disposed to be collinear with a line passing in the direction of gravity through the axis c1 of rotation of the radiation emitter 210.

According to an embodiment, the fourth weight adders 2320 and 2320a can further include a weight installing member 2321 installed in the outer housing of the radiation emitter 210, and a weight 2323 installed in the weight installing member 2321 and adding an additional weight to the radiation emitter 210. The weight installing member 2321 can include resting space 2322 in which the weight 2323 is inserted, and further include a cover (not shown) to open or close the resting space 2322, as necessary.

If the weight 2323 is installed in the weight installing member 2321, the fourth weight adders 2320 and 2320a can apply a predetermined force to the radiation emitter 210 and the second support 220 in the direction of gravity, and accordingly, the predetermined force can also be applied to the cable, wire, belt, or chain installed in the inside of the post frame 2220. The predetermined force applied to the cable, wire, belt, or chain can exceed a friction force between the pulley or toothed wheel in the carriage 2250 and the surface on which the pulley or toothed wheel is located, or a support force provided by the spring balancer, and accordingly, the cable, wire, belt, or chain can move in the application direction of the predetermined force. As a result, the plurality of posts 2221 to 2225 of the post frame 2220 can extend so that the radiation emitter 210 can move automatically in the down direction M21, as shown in FIG. 28. As described above, since the fourth weight adders 2320 and 2320a are collinear with the line passing in the direction of gravity through the axis c1 of rotation of the radiation emitter 210, the radiation emitter 210 can not rotate in a specific direction.

According to embodiments, a single fourth weight adder 2320 and 2320a or two or more fourth weight adders 2320 and 2320a can be provided.

According to embodiments, the fourth weight adder 2320 and 2320a can be configured to install the weight 2323 in the outer housing of the radiation emitter 210 using magnetism, or to install the weight 2323 in the outer housing of the radiation emitter 210 using a loop or hook. Also, the fourth weight adder 2320 and 2320a can be configured to install the weight 2323 in the outer housing of the radiation emitter 210 using various methods that can be considered by a designer.

According to another embodiment, the fourth weight adder 2320 can be formed in the second support 220, and more specifically, the fourth weight adder 2320 can be formed in one end of the second support 220. In this case, in the end of the second support 220, a predetermined weight installing member, which a weight can be inserted into, caught by, or attached to using magnetism can be provided.

The second brake 250 can put a brake on the radiation emitter 210 so as for the radiation emitter 210 to rotate no longer in the predetermined direction r20. For example, the second brake 250 can be installed in the shaft member coupled with the outer housing of the radiation emitter 210 and/or the motor coupled with the shaft member to stop the shaft member or the motor, thereby preventing the radiation emitter 210 from rotating with respect to a predetermined axis c1 of rotation. The second brake 250 can stop the shaft member using a friction, a magnetic field, and/or the pressure of air or fluid. Also, various braking means for stopping the shaft member can be adopted as the second brake 250. However, the second brake 250 can be omitted, according to another embodiment.

The support brake 251 can stop extending and/or shortening the length of the second support 220. The support brake 251 can be installed in the moving carriage 2250 to prevent at least one rotary body from rotating, and accordingly stop the cable interlocked with the rotary body. Accordingly, one end 2201 of the post frame 2220 can stop moving in the up direction or in the down direction, and the second support 220 can also stop extending or being shortened. The support brake 251 can stop the rotary body using a friction force of a disc or drum, a magnetic field, or the pressure of air or fluid. Also, various braking means for stopping the rotary body can be used as the support brake 251. However, the support brake 251 can be omitted, according to another embodiment.

The position detector 241 can be provided to acquire information for measuring a position of the radiation emitter 210 on the z-axis. More specifically, the position detector 241 can acquire information for measuring a position of the radiation emitter 210 on the z-axis, generate an electrical signal corresponding to the information, and transfer the electrical signal to at least one of the driving controller 290 and the controller 310 of the control apparatus 300.

According to an embodiment, the position detector 241 can sense a height of the radiation emitter 210 from the ground. In this case, the position detector 241 can be implemented with a distance sensor installed on the lower surface of the radiation emitter 210 or the end 2201 of the post frame 2220. The distance sensor can use electromagnetic waves of visible light, infrared light, etc., or ultrasonic waves. The distance sensor can irradiate electromagnetic waves or ultrasonic waves toward the ground, receive the electromagnetic waves or ultrasonic waves reflected from the ground, generate an electrical signal corresponding to the received electromagnetic waves or ultrasonic waves, and transfer the electrical signal to at least one of the driving controller 290 and the controller 310 of the control apparatus 300. The at least one of the driving controller 290 and the controller 310 can acquire information about a time at which the electromagnetic waves or ultrasonic waves are reflected and return, and measure a distance between the radiation emitter 210 and the ground based on the information about the time to thus measure a position of the radiation emitter 210.

According to another embodiment, the position detector 241 can detect an extending length of the second support 220. In this case, the position detector 241 can be implemented as communication modules respectively installed on both ends 2201 and 2202 of the second support 220. At least one of the communication modules, the driving controller 290, and the controller 310 can sense the extending length of the second support 220 using the intensities of signals transmitted/received to/from each other through the communication modules.

According to another embodiment, the position detector 241 uses a rotary encoder or the like to measure the number of revolution, the speed of rotation, and/or the direction of rotation of the rotary body installed in the moving carriage 2250. The number of revolution, the speed of rotation, and/or the direction of rotation, measured by the position detector 241 can be transferred to at least one of the driving controller 290 and the controller 310, and the at least one of the driving controller 290 and the controller 310 can measure the extending length of the second support 220 based on the number of revolution, the speed of rotation, and/or the direction of rotation.

Also, various means for detecting a position of the radiation emitter 210 on the z-axis can be used as the position detector 241.

The rotation angle detector 242 can acquire information about the angle of rotation, the number of revolution, and/or the direction of rotation of the shaft member coupled with the radiation emitter 210, such as the rotary encoder. The rotation angle detector 242 can transfer the acquired information in the form of an electrical signal to at least one of the driving controller 290 and the controller 310. The at least one of the driving controller 290 and the controller 310 can decide what degree of angle the radiation emitter 210 rotates to, according to the result of the sensing by the rotation angle detector 242. The rotary encoder can be implemented by adopting, for example, at least one of an optical incremental encoder, an optical absolute encoder, a magnetic encoder, and/or a resolver. Also, the rotation angle detector 242 can be implemented as a potentiometer as described above.

The driving controller 290 controls overall operations of the radiation irradiation apparatus 200, and process various information required by the radiation irradiation apparatus 200. The driving controller 290 can be implemented as a processor composed of at least one semiconductor chip and the related components. The processor is configured to execute one or more programs.

For example, the driving controller 290 can control driving of the radiation tube 2101. More specifically, the driving controller 290 can transfer a control signal to a power source 12, the radiation tube 2101, or a switch device (not shown) for connecting or cutting off them, at a specific time to apply electricity of a predetermined voltage to the radiation tube 2101 so that the radiation tube 2101 can emit radiation. According to an embodiment, the driving controller 290 can generate a control signal just after the radiation emitter 210 stops rotating or the second support 220 stops extending or being shortened according to operation of the second brake 250 or the support brake 251, or when a predetermined time period elapses after the radiation emitter 210 stops rotating or the second support 220 stops extending or being shortened, and transfer the control signal to the radiation tube 2101, the power source 212, or the switch device. According to an embodiment, if the radiation emitter 210 is stopped according to operation of the second brake 270, the driving controller 290 can generate a control signal immediately or when a predetermined time period elapses, and transfer the control signal to the radiation tube 2101, the power source 212, or the switch device. Accordingly, the radiation emitter 210 can irradiate radiation onto a subject after the radiation emitter 210 stops moving and/or after the radiation detector 110 stops moving.

According to another example, the driving controller 290 generates a control signal related to operations of the second driver 250 and the support driver 251, based on an electrical signal transferred from the position detector 241 and/or the rotation angle detector 242. According to an embodiment, the driving controller 290 can determine a rotation angle of the radiation emitter 210 according to the result of sensing by the rotation angle detector 242, and generate a control signal for the second brake 250 based on the rotation angle. In this case, the driving controller 290 can compare the rotation angle to information about a predetermined scanning direction, that is, information about an irradiation angle, and then if the driving controller 290 determines that the rotation angle is identical to the irradiation angle, the driving controller 290 generates a brake signal for the second brake 250. According to another embodiment, the driving controller 290 decides a position of the radiation emitter 210 on the z-axis according to the result of sensing by the position detector 241, and generates a brake signal for the support brake 251 according to the result of the decision. In this case, the driving controller 290 can compare the decided position of the radiation emitter 210 to a predetermined scanning position, that is, a position on the z-axis for irradiating ration, and if the driving controller 290 determines that the decided position is identical to the predetermined scanning position, the driving controller 290 can generate a brake signal for the support brake 251.

The driving controller 290 of the radiation irradiation apparatus 200 can control the radiation emitter 210 to be located at a position corresponding to the position of the radiation detector 110, and/or can control the radiation emitter 210 to be tilted. For example, the driving controller 290 can receive information about the position of the radiation detector 110 from the radiation detection apparatus 100 and/or the controller 310 of the control apparatus 300, decide a position of the radiation emitter 210 and/or a radiation irradiation angle of the radiation emitter 210 based on the received information, and control the radiation emitter 210 according to the decided position.

The radiation detector 110 can move in the down direction M11 by the weight of the radiation detector 110 and/or an additional weight provided by the first weight adder 130. If the radiation emitter 210 rotates in the predetermined direction r22 by the third weight adder 2311 according to the first embodiment so that the radiation irradiation surface of the radiation emitter 210 is tilted to face the down direction M11, the driving controller 290 can control the radiation emitter 210 to stop rotating at an angle at which radiation can be properly incident to the radiation detector 110. This operation can be applied in the same manner or through slight modification to the case in which the radiation detector 110 moves in the up direction M12 by an additional weight provided by the second weight adder 160, and the radiation emitter 210 rotates in the opposite direction r23 by the third weight adder 2315 according to the second embodiment so that the radiation irradiation surface of the radiation emitter 210 is tilted to face the up direction.

Also, when the fourth weight adder 2320 adds an additional weight to the radiation emitter 210 and/or the second support 220, likewise, the driving controller 290 can control the second support 220 to move the radiation emitter 210 to a position at which radiation can be properly incident to the radiation detector 110.

The driving controller 290 may be omitted according to embodiments. If the driving controller 290 is omitted, the driving controller 190 of the radiation detection apparatus 100 and/or the controller 310 of the control apparatus 300 can perform operations of the driving controller 290.

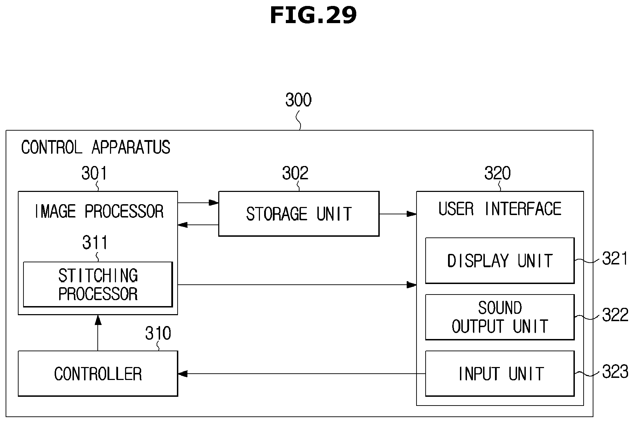

Hereinafter, the control apparatus 300 will be described in more detail with respect to FIG. 29.

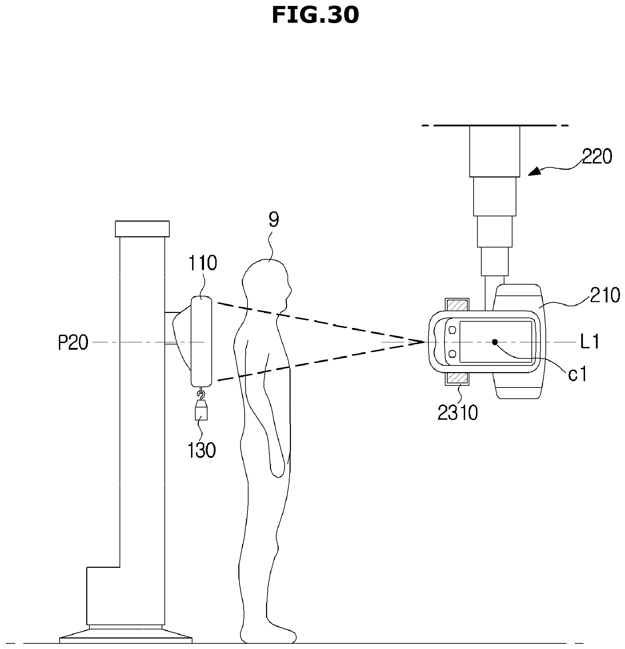

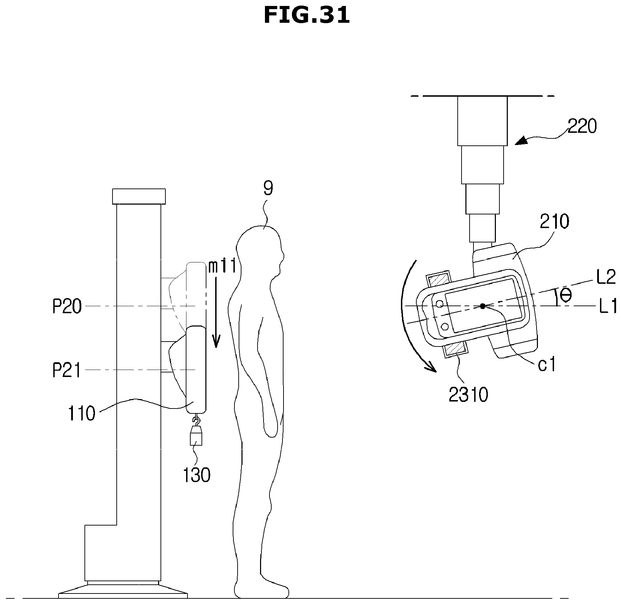

FIG. 29 is a block diagram of a control apparatus according to an embodiment of the present disclosure, FIG. 30 shows an example of an acquired radiation image, and FIG. 31 shows an example of a synthesized radiation image.

The control apparatus 300 can include an image processor 301, a storage unit 302, a controller 310, and a user interface 320, as shown in FIG. 29.

The image processor 301 can acquire a radiation image based on an electrical signal corresponding to radiation received by the radiation detector 110. In this case, the image processor 301 can produce a plurality of radiation images i1, i2, and i3, as shown in FIG. 30. Also, the image processor 301 can perform post processing on the radiation images i1, i2, and i3 to correct the radiation images i1, i2, and i3.

According to an embodiment, the image processor 301 includes a stitching processor 310. The stitching processor 310 can synthesize the plurality of radiation images i1, i2, and i3 acquired by photographing the subject at different locations to thus produce at least one radiation image i10 (also, referred to as a stitched image i10) as shown in FIG. 31. In this case, the stitching processor 310 can overlap areas i11 and i12 appearing in common in the plurality of radiation images i1, i2, and i3 to produce an image i10. The stitching processor 310 can detect feature points such as edges from the plurality of radiation images i1, i2, and i3 for stitching processing, and compare the plurality of radiation images i1, i2, and i3 to each other using the detected feature points to detect areas i11 and i12 appearing in common in the plurality of radiation images i1, i2, and i3, and then overlap the areas i11 and i12 to produce a radiation image i10. Also, the stitching processor 310 can synthesize the plurality of radiation images i1, i2, and i3 using one of various well-known image registration methods to acquire a radiation image i10. According to an embodiment, the stitching processor 310 corrects the synthesized image i10. For example, the stitching processor 310 can compensate the curvature of the image i10 to correct distortion of the image i10.

The image processor 301 can be implemented with one or more semiconductor chips and the related components. For example, the image processor 301 can be implemented as a graphic processing unit GPU. According to an embodiment, the controller 310 can function as the image processor 301.

The storage unit 302 can store the radiation images i1, i2, i3, and i10 produced by the image processor 301 temporarily or non-temporarily. Also, the storage unit 302 can store various kinds of programs related to operations of the image processor 301 and/or the controller 310. The storage unit 302 can be implemented with a magnetic drum, a magnetic disk, an optical disk, and/or a semiconductor chip.

The controller 310 can control overall operations of the radiographic imaging apparatus 1. The controller 310 can generate a predetermined control signal based on an electrical signal received from an input unit 323 of a user interface 320, and transfer the predetermined control signal to at least one of the radiation detection apparatus 100 and the radiation irradiation apparatus 200 so that the at least one of the radiation detection apparatus 100 and the radiation irradiation apparatus 200 can operate according to a user's requirement. The controller 310 can perform operation of the driving controller 190 or 290 described above, as necessary. For example, the controller 310 can receive an electrical signal from the position detector 140 or 241 or the rotation angle detector 242, generate a control signal based on the received electrical signal, and transfer the control signal to the first brake 170 and/or the second brake 250. The first brake 170 and/or the second brake 250 can operate according to the received control signal, and stop movement or rotation of the radiation detector 110 and/or the radiation emitter 210.

The image processor 301 and/or the controller 310 can be implemented as a processor composed of at least one semiconductor and the related components. In this case, the image processor 301 and the controller 310 can be implemented as separate processors or a single processor.

The user interface 320 can provide a user with various kinds of information or receive various commands from the user. The user interface 320 can include, for example, a display 321, a sound output unit 322, and an input unit 323. However, some of the above-mentioned components can be omitted. The user interface 320 can further include various input/output means for providing information to the user or receiving commands from the user, according to an embodiment. For example, the user interface 320 can further include a light emitting diode (LED) light. In FIG. 29, an example in which the user interface 320 is included in the control apparatus 300 is shown. However, the installation location of the user interface 320 is not limited to the control apparatus 300. The user interface 320 can be installed in the radiation irradiation apparatus 200 or the radiation detection apparatus 100, as necessary. In this case, a part (for example, the sound output unit 322) of the user interface 320 can be installed in any one of the radiation detection apparatus 100 and the radiation irradiation apparatus 200, and the other part (for example, the display 321 and the input unit 323) of the user interface 320 can be installed in the control apparatus 300.

The display 321 can display the acquired radiation images i1, i2, and i3 and the stitched image i10 for the user. The display 321 can display various kinds of information related to operations of the radiographic imaging apparatus 1, as necessary. For example, the display 321 can display various information including the position of the radiation detector 110 and/or the radiation emitter 210, a degree of tilting of the radiation emitter 210, or whether or not the radiation emitter 210 irradiates radiation, and the like. According to an embodiment, the display 321 can display information about whether at least one of the first weight adder 130, the second weight adder 160, the third weight adder 2310, and the fourth weigh adder 2320 applies an additional weight to the radiation detector 110, the radiation emitter 210, and/or the second support 220. In this case, the radiation detection apparatus 100 and the radiation irradiation apparatus 200 can further include a weight sensor (not shown) to determine whether an additional weight is applied. Also, according to an embodiment, the display 321 can display an image of a predetermined pattern to inform the user of whether the radiation detector 110 and the radiation emitter 210 are located at appropriate scanning positions.

The sound output unit 322 can output predetermined sound to provide the predetermined sound to the user. Herein, the predetermined sound can include beep sound or voice. According to an embodiment, the sound output unit 322 can output sound of a predetermined pattern to help the user check whether the radiation detector 110 and/or the radiation emitter 210 is located at an appropriate position.

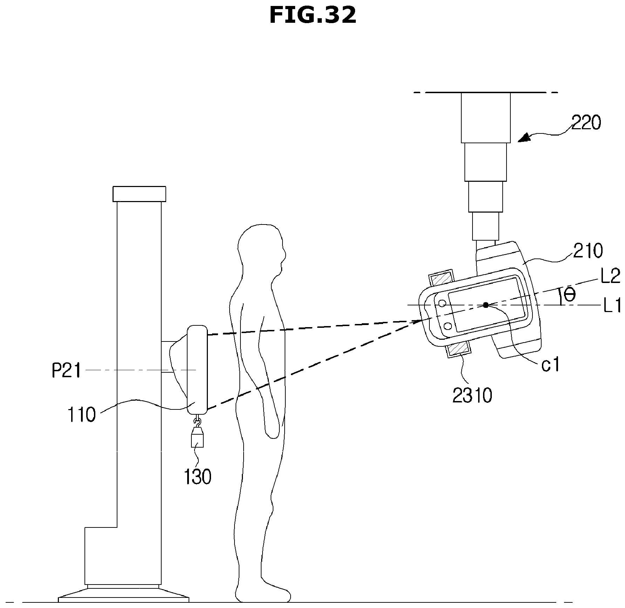

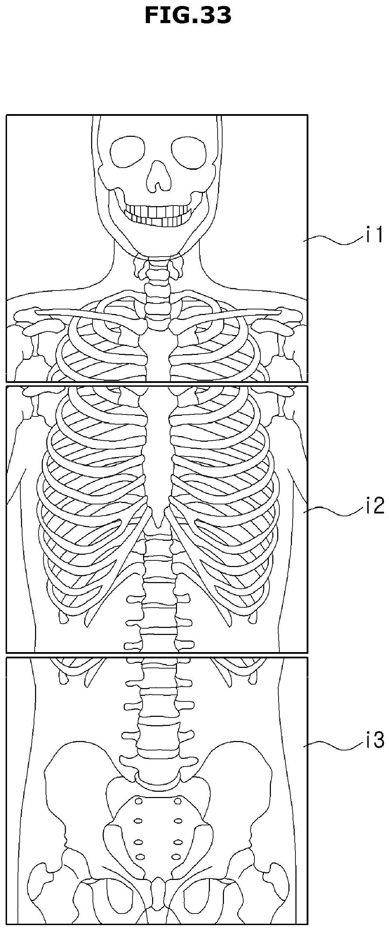

FIG. 32 is a view for describing a process for setting an initial position of at least one of a radiation detector and a radiation emitter, and FIG. 33 is a view for describing a pattern of sound that is output from a sound output unit when at least one of a radiation detector and a radiation emitter approaches a scanning position.

For example, as shown in FIG. 32, when the radiation detector 110 is located at an initial position p11 spaced by a predetermined distance from a scanning position p15 being an appropriate position for radiography, a user can move the radiation detector 110 manually or automatically from the initial position p11 to the scanning position p15. In this case, the moved position detector 140 of the radiation detection apparatus 100 can sense the positions p11 to p15 of the radiation detector 110, and transfer an electrical signal corresponding to the results of the sensing to any one of the driving controller 190 and the controller 310. The driving controller 190 or the controller 310 can decide a position p11 to p15 of the radiation detector 110 based on the electrical signal corresponding to the result of the sensing, generate a control signal corresponding to the result of the decision, and transfer the control signal to the sound output unit 322. The sound output unit 322 can output sound of a predetermined pattern based on the control signal transferred from the driving controller 190 or the controller 310.

The sound output from the sound output unit 322 can change depending on the position p11 to p15 of the radiation detector 110. More specifically, as shown in FIG. 33, if the radiation detector 110 is located at the initial position p11, the radiation detector 110 can output sound according to a first pattern t1. For example, the radiation detector 110 can output sound such as beep sound at relatively long time intervals, for a relatively long time period.

If the radiation detector 110 approaches the scanning position p15 from the initial position p11, the sound output unit 322 can output sound of different patterns, that is, second to fourth patterns t2 to t4, whenever the radiation detector 110 arrives at the specific positions p12 to p14. For example, as the radiation detector 110 approaches the scanning position p15, the sound output unit 322 can output sound repeatedly for a relatively shorter time period. In this case, the interval at which the sound output unit 322 outputs sound repeatedly can also be relatively further shortened.

If the radiation detector 110 arrives at the scanning position p15, the sound output unit 322 can output sound in a fifth pattern t5 that is different from the first to fourth patterns t1 to t4. For example, the sound output unit 322 can output sound without any interruption, as shown in FIG. 33.

The sound output patterns t1 to t5 of the sound output unit 322 according to the positions p11 to p15 of the radiation detector 110 can be arbitrarily decided according to a designer's selection.

The embodiment in which the sound output unit 322 outputs sound in the different patterns t1 to 5 according to the positions p11 to p15 of the radiation detector 110 can be applied to an embodiment in which the sound output unit 322 outputs sound of a different pattern according to the position or rotation angle of the radiation emitter 210, in the same way or through slight modification.

According to an embodiment, in order for a user to check whether the radiation detector 110 and/or the radiation emitter 210 is located at an appropriate scanning position or inclined at an appropriate angle, the display 321, instead of the sound output unit 322, can display a predetermined image according to the position p11 to p15 of the radiation detector 110, and the position or rotation angle of the radiation emitter 210. Also, light means such as a LED light, instead of the sound output unit 322, can flicker on/off in a predetermined pattern according to the position p11 to p15 of the radiation detector 110, and the position or rotation angle of the radiation emitter 210.

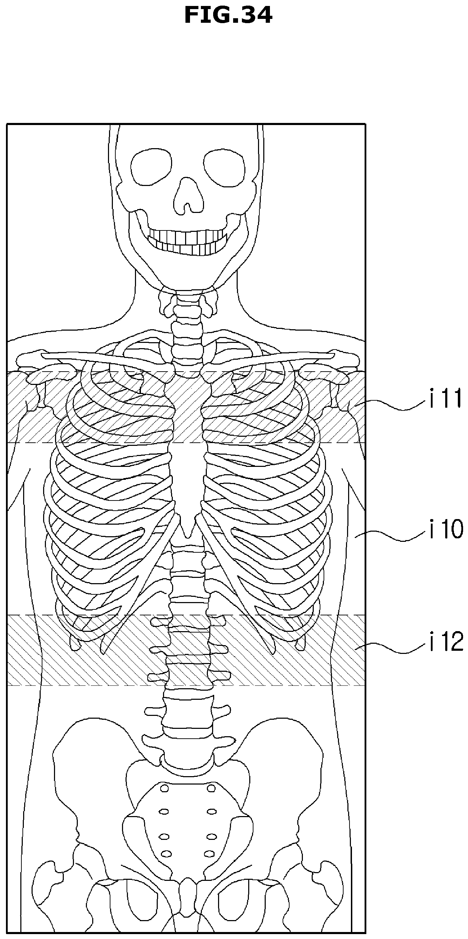

Hereinafter, a method of controlling the radiographic imaging apparatus described above, according to various embodiment of the present disclosure, will be described with reference to FIGS. 34 to 39.

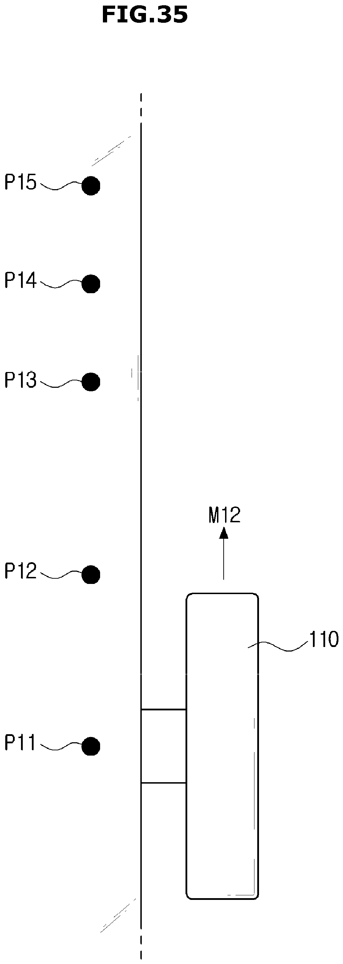

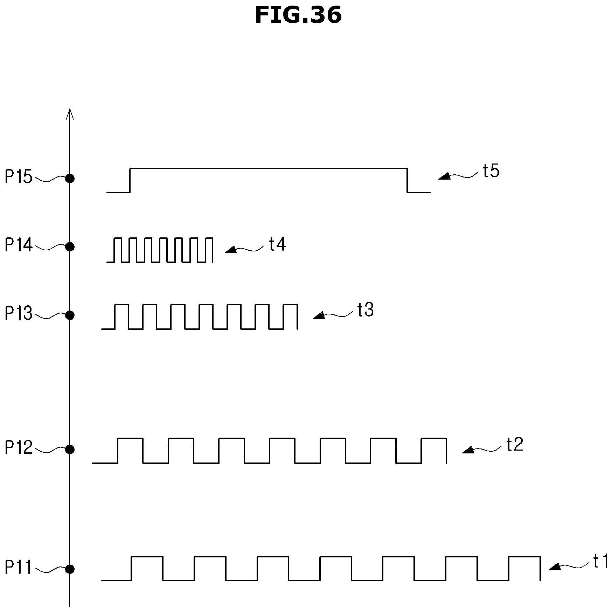

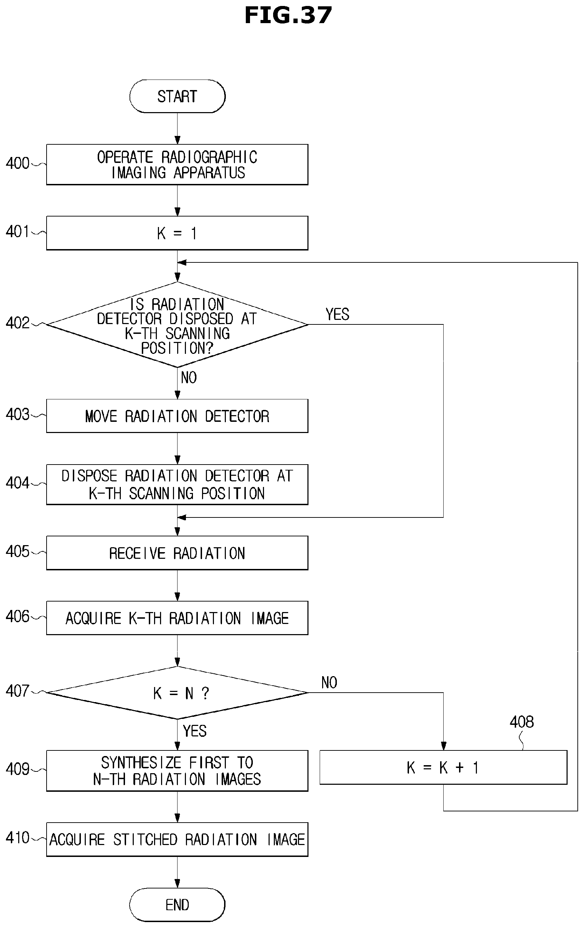

FIG. 34 is a first flowchart illustrating a method of controlling a radiographic imaging apparatus, according to an embodiment of the present disclosure. More specifically, FIG. 34 is a flowchart illustrating a method of controlling a radiation detection apparatus according to an embodiment of the present disclosure. FIG. 35 is a first view for describing a process in which the radiographic imaging apparatus performs stitching scanning, FIG. 36 is a second view for describing a process in which the radiographic imaging apparatus performs stitching scanning, and FIG. 37 is a third view for describing a process in which the radiographic imaging apparatus performs stitching scanning.

As shown in FIG. 34, if the radiographic imaging apparatus 1 starts operating according to a user's manipulation or a predetermined setting, in operation 400, it can be determined whether the radiation detector 110 of the radiographic imaging apparatus 200 is located at a position for initially performing radiation scanning, that is, at a first scanning position p20, in operations 401 and 402. The determination on whether the radiation detector 110 is located at the first scanning position p20 can be performed by the driving controller 190 or the controller 310, based on a signal output from the position detector 140.

If it is determined that the radiation detector 110 is not located at the first scanning position p20 ("No" in operation 402), the radiation detector 110 can move to the first scanning position p20, in operation 403. In this case, the movement of the radiation detector 110 can be performed by the user's manual manipulation, by the first weight adder 130 attached on the radiation detector 110 and the weight of the radiation detector 110, or by the second weight adder 160.

According to an embodiment, if the radiation detector 110 is moved by the user's manual manipulation, the user interface 320 can display whether the position of the radiation detector 110 is identical to the first scanning position p20, or how close the position of the radiation detector 110 is to the first scanning position p20, for the user, using sound of the predetermined patterns t1 to p5, an image, or flickering on/off of light, etc.

If the radiation detector 110 moves to arrive at the first scanning position p20, the first brake 170 of the radiation detection apparatus 100 can stop the radiation detector 110 at the first scanning position p20, in operation 404.

If the radiation detector 110 is located at the first scanning position p20 ("Yes" in operation 402), the radiation detector 110 can move no longer and stop. The radiation detector 110 can be maintained at the first scanning position p20, according to operation of the first brake 170.

As shown in FIG. 35, the radiation detector 110 located at the first scanning position p20 can receive radiation irradiated from the radiation emitter 210 to thus acquire an electrical signal corresponding to the radiation, in operation 405.

The radiographic imaging apparatus 1 can acquire a first radiation image based on the electrical signal corresponding to the received radiation, and store the first radiation image temporarily or non-temporarily in the storage unit 302, in operation 406. In this case, the first radiation image can be acquired by the image processor 301 of the control apparatus 300.

If the radiographic imaging apparatus 1 is set to acquire N radiation images at N positions, and N is greater than 1 ("No" in operation 407), the radiographic imaging apparatus 1 can again perform the above-described operations 402 to 406 to thus further acquire a second radiation image, in operation 408.

In this case, first, the driving controller 190 and/or the controller 310 can determine whether the radiation detector 110 is located at a second scanning position p21 for acquiring the second radiation image, in operation 402.

If the radiation detector 110 is not located at the second scanning position p21 ("No" in operation 402), the first brake 170 can be released, and the radiation detector 110 can move to the second scanning position p21 by the first weight adder 130 and the weight of the radiation detector 110, or by the second weight adder 160. For example, the radiation detector 110 can move in the down direction M12 by the first weight adder 130 and the weight of the radiation detector 110, in operation 403, as shown in FIG. 36.

If the radiation detector 110 arrives at the second scanning position p21, the first brake 170 can operate automatically to stop the radiation detector 110 at the second scanning position p21 so that the radiation detector 110 is disposed at the second scanning position p21, in operation 404.

As shown in FIG. 37, the radiation detector 110 disposed at the second scanning position p21 can receive radiation irradiated from the radiation emitter 210, and again acquire an electrical signal corresponding to the radiation, in operation 405. In this case, the radiation emitter 210 can also move or rotate in order to irradiate radiation in a direction in which the radiation detector 110 is disposed.

The radiographic imaging apparatus 1 can acquire a second radiation image based on the electrical signal corresponding to the received radiation, and store the second radiation image, in operation 406.

The driving controller 190 and/or the controller 310 of the radiographic imaging apparatus 1 can again determine whether N radiation images are acquired from the N positions, and if the driving controller 190 and/or the controller 310 determines that N radiation images are not acquired from the N positions ("No" in operation 407), the driving controller 190 and/or the controller 310 can perform the above-described operations 402 to 408 repeatedly to acquire N radiation images. In this case, the radiation detector 110 can move sequentially from the third scanning position to the N-th scanning position (not shown) by the first weight adder 130 and the weight of the radiation detector 110 to acquire electrical signals corresponding to incident radiation at the respective first to N-th scanning positions. That is, the image processor 301 can acquire a third radiation image to a N-th radiation image respectively corresponding to the third scanning position to the N-th scanning position.

If the first to N-th radiation images are acquired as described above, the image processor 301 can combine the first to N-th radiation images, as shown in FIG. 25, in operation 409, to acquire a stitched radiation image, in operation 410.

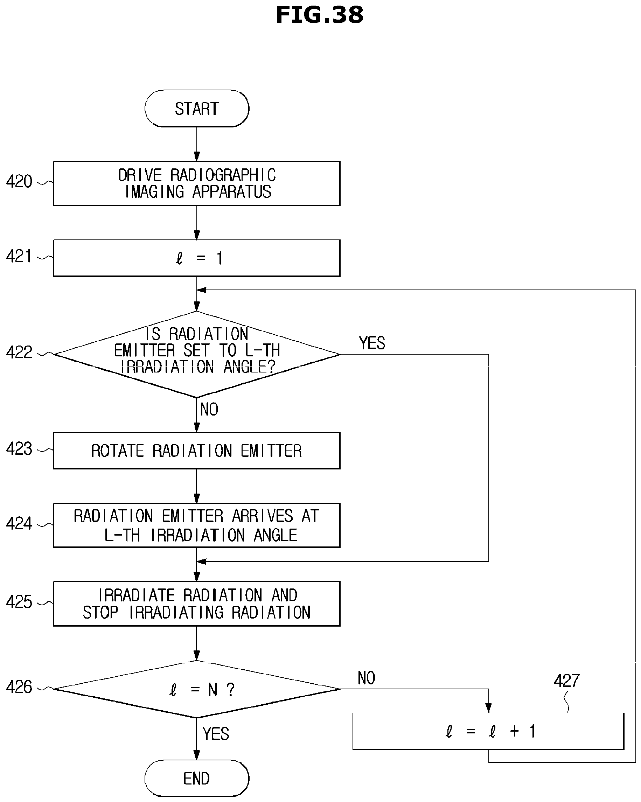

FIG. 38 is a second flow chart illustrating a method of controlling a radiographic imaging apparatus, according to an embodiment of the present disclosure. More specifically, FIG. 38 is a flowchart illustrating a method of controlling a radiation irradiation apparatus, according to an embodiment of the present disclosure.

As shown in FIG. 38, if the radiographic imaging apparatus 1 is driven, in operation 420, it can be determined whether the angle of the radiation emitter 210 of the radiographic imaging apparatus 200 is set to a first irradiation angle, in operations 421 and 422. The determination on whether the angle of the radiation emitter 210 is set to the irradiation angle can be performed by the driving controller 290 or the controller 310 based on a signal output from the rotation angle detector 242 of the radiation irradiation apparatus 200. The first irradiation angle can be set to correspond to the position p20 of the radiation detector 110. More specifically, the first irradiation angle can be set to locate the radiation detector 110 in a radiation irradiation direction L1 of the radiation emitter 210. In other words, the first irradiation angle can be set to locate the first scanning position p20 in the radiation irradiation direction L1 of the radiation emitter 210. The first irradiation angle can include the case in which an included angle formed between the ground and the radiation irradiation direction L1 is 0 degree.

If the angle of the radiation emitter 210 is not set to the first irradiation angle, the radiation emitter 210 can be rotated by a user's manual manipulation, or by the third weight adder 2310 attached on the radiation emitter 210, in operation 423. In the latter case, for example, the radiation emitter 210 can rotate such that the light irradiation surface faces the down direction by the third weight adder 2311 of the first embodiment.
If the radiation emitter 210 is rotated by the user's manual manipulation, the user interface 320 can provide the user with information about whether the irradiation angle of the radiation emitter 210 is identical to the first irradiation angle or how close the irradiation angle of the radiation emitter 210 is to the first irradiation angle, using the predetermined patterns t1 to t5.
If the radiation emitter 210 is rotated by a predetermined rotation angle to arrive at the first irradiation angle, the second brake 250 of the radiation emitter 210 can operate to prevent the radiation emitter 210 from rotating, in operation 424.

If the radiation emitter 210 reaches the first irradiation angle, the radiation emitter 210 can generate radiation of predetermined energy according to the control of the driving controller 290 and/or the controller 310, and then irradiate the radiation to the radiation detector 110, in operation 425, as shown in FIG. 35. After the radiation emitter 210 irradiates radiation for a predetermined time period, the radiation emitter 210 can stop irradiating radiation. As described above, the radiation detector 110 can receive radiation transmitted through a subject 9, and the image processor 301 can produce a radiation image corresponding to the received radiation.

Operation 423 in which the radiation emitter 210 rotates to the first irradiation angle and/or operation 424 in which the radiation emitter 210 stops at the first irradiation angle can be performed simultaneously with, earlier than, or later than operation 403 in which the radiation detector 110 moves to the first scanning position p20 and/or operation 404 in which the radiation detector 110 stops at the first scanning position p20. In other words, the radiation detector 110 and the radiation emitter 210 can operate simultaneously, the radiation emitter 210 can rotate after the radiation detector 110 moves, or the radiation detector 110 can move after the radiation emitter 210 rotates.

If the radiographic imaging apparatus 1 is set to acquire N radiation images corresponding to N positions (N>1), the radiation irradiation apparatus 200 can perform the above-described operations 422 to 425 repeatedly until the N radiation images are acquired, in operation 427.

More specifically, the driving controller 190 of the radiation irradiation apparatus 100 and/or the controller 310 of the control apparatus 300 can determine whether the radiation emitter 210 is set to a second irradiation angle for acquiring a second radiation image, in operation 422.

If the radiation emitter 210 is not at the second irradiation angle ("No" in operation 422), the radiation emitter 210 can be released from its stopped state by the second brake 250, and rotate to the second irradiation angle by the third weight adder 2310, in operation 423. For example, as shown in FIG. 36, the radiation emitter 210 can be rotated by the third weight adder 2311 of the first embodiment.

If the radiation emitter 210 is rotated by a predetermined angle θ to arrive at the second irradiation angle, the irradiation detection direction L2 of the radiation emitter 210 can face the second position p21 that is relatively lower than the first position p20. If the radiation emitter 210 arrives at the second irradiation angle, the second brake 250 can operate automatically according to a predetermined setting or according to the control of the driving controller 290 and/or the controller 310 so as to enable the radiation emitter 210 to stop at the second irradiation angle, in operation 424.

As shown in FIG. 36, the radiation emitter 110 positioned at the second irradiation angle can again irradiate radiation to the radiation detector 110 immediately according to a predetermined setting or after a predetermined time period elapses, in operation 425, and the radiation detector 110 can receive radiation irradiated from the radiation emitter 210 and again acquire an electrical signal corresponding to the radiation.

The driving controller 190 and/or the controller 310 can again determine whether N radiation images are acquired at N positions. If the driving controller 190 and/or the controller 310 determines that N radiation images are not acquired at the N positions ("No" in operation 426), the driving controller 190 and/or the controller 310 can perform the above-described operations 422 to 425 repeatedly so as to control the radiation emitter 210 to irradiate radiation at least N times. In this case, the radiation emitter 210 can be rotated sequentially to the third to N-th irradiation angles (not shown) by the additional weight applied by the third weight adder 2310, and irradiate radiation at the third to N-th irradiation angles.

FIGS. 35 to 37 show an embodiment in which the radiation detector 110 moves in the down direction, and the radiation emitter 210 is tilted such that the radiation irradiation surface faces the down direction, however, the embodiment can be applied to an embodiment in which the radiation detector 110 moves in the up direction and the radiation emitter 210 is tilted such that the radiation irradiation surface faces the up direction, in the same way or through slight modification.

The operations of the radiographic imaging apparatus 1, as shown in FIG. 38, can be applied to the case in which the radiation emitter 210 is moved in the down direction or in the up direction by the weight of the radiation emitter 210 and/or the fourth weight adder 2320 of the second support 220, in the same way. In this case, the radiation emitter 210 can irradiate radiation to the radiation detector 110 multiple times at a plurality of heights. The plurality of heights can correspond to the positions of the radiation detector 110. According to an embodiment, the plurality of heights can be equal to or approximate the heights of the radiation detector 110 from the ground.

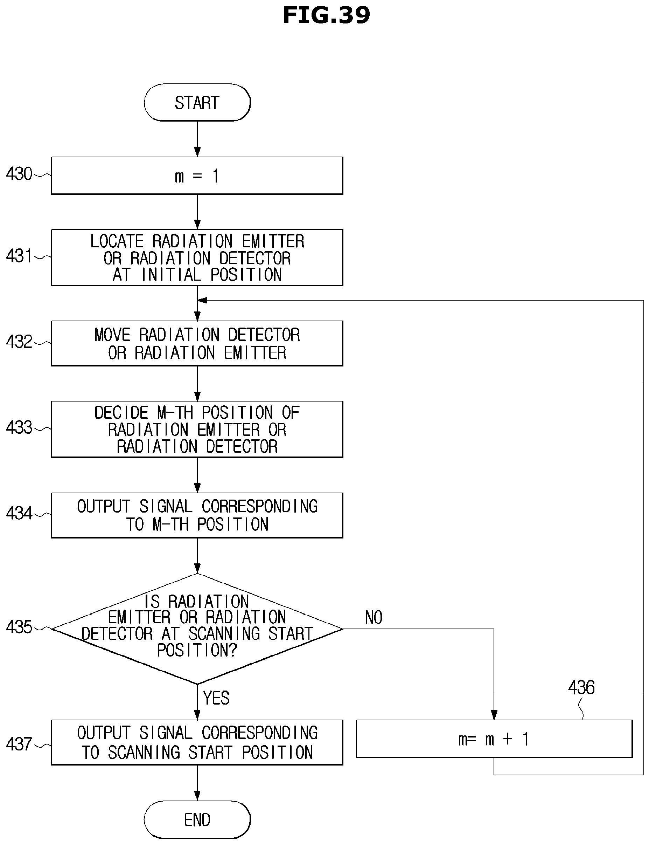

FIG. 39 is a flowchart illustrating a method of setting the initial positions of a radiation emitter and a radiation detector, according to an embodiment of the present disclosure, in a method of controlling a radiographic imaging apparatus.

As shown in FIG. 39, the radiation detector 110 can be located at an initial position, wherein the initial position of the radiation detector 110 can be different from a scanning position for radiography, in operation 431. The initial position can be lower than or higher than the scanning position. Also, the radiation emitter 210 can be located at an initial position. The initial position of the radiation emitter 210 can include an irradiation angle to which the radiation emitter 210 is first set to, a height at which the radiation emitter 210 is first located, and both of the irradiation angle and the height.

The radiation detector 110 or the radiation emitter 210 can move from the initial position to a first position, in operation 432. In this case, the radiation detector 110 or the radiation emitter 210 can be moved by a user's manual manipulation. The radiation detector 110 can be moved by operation of the detector moving member 150 of the radiation detection apparatus 100. Also, the radiation emitter 210 can be moved by the motor installed in the emitter coupling housing 2210 or when the second support 220 extends or is shortened.
After the radiation detector 110 or the radiation emitter 210 moves, it can be determined whether the radiation detector 110 or the radiation emitter 210 arrives at the first position, in operation 433. The position of the radiation detector 110 or the radiation emitter 210 can be decided using the position detector 140 or 241. The driving controller 190 or 290 or the controller 310 can compare information about the first position to a current position of at least one of the radiation detector 110 and the radiation emitter 210, and determine whether the at least one of the radiation detector 110 and the radiation emitter 210 arrives at the first position, according to the result of the comparison. According to an embodiment, the radiation emitter 210 can rotate, and in this case, a rotation angle of the radiation emitter 210 can be decided using the rotation angle detector 242, and the driving controller 190 or 290 or the controller 310 can determine whether the rotation angle is identical to a first angle.

If the driving controller 190 or 290 or the controller 310 determines that the radiation detector 110 or the radiation emitter 210 arrives at the first position or that the radiation emitter 210 rotates to the first angle, the driving controller 190 or 290 or the controller 310 can generate a control signal corresponding to the position of the radiation detector 110 or the radiation emitter 210 or the rotation angle of the radiation emitter 210, and transfer the generated control signal to the user interface 320. In this case, the control signal can include information about at least one of a sound output pattern, an image display pattern, and a flickering on/off pattern of a light, corresponding to the position or rotation angle.

At least one of the display unit 321, the sound output unit 321, and the light of the user interface 320 can operate according to a control signal transferred from the driving controller 190 or 290 or the controller 310, and output a signal corresponding to the first position of the radiation detector 110 or the radiation emitter 210 or the first angle of the radiation emitter 210 to the outside, in operation 434. In this case, the display 321 can display a predetermined image according to the first position of the radiation detector 110 or the radiation emitter 210 or the first angle of the radiation emitter 210. The sound output unit 321 can output sound in a predetermined pattern as shown in FIG. 27, according to the first position of the radiation detector 110 or the radiation emitter 210 or the first angle of the radiation emitter 210. Also, the light can flash on/off in a predetermined pattern according to the first position of the radiation detector 110 or the radiation emitter 210 or the first angle of the radiation emitter 210.

Successively, it can be determined whether the first position of the radiation detector 110 or the radiation emitter 210 or the first rotation angle of the radiation emitter 210 is a scanning start position or a radiation irradiation angle, in operation 435.

If the first position of the radiation detector 110 or the radiation emitter 210 is not the scanning start position ("No" in operation 435), the radiation detector 110 or the radiation emitter 210 can continue to move automatically or manually. Also, if the first rotation angle of the radiation emitter 210 is not the first rotation angle, the radiation emitter 210 can continue to rotate automatically or manually, in operation 432.

If the radiation detector 110 or the radiation emitter 210 moves to a second position, or if the radiation emitter 210 rotates to a second rotation angle from the first rotation angle, in operations 432 and 433, at least one of the display 321, the sound output unit 322, and the light of the user interface 320 can output a signal of a pattern corresponding to the second position and/or the second rotation angle, in operation 434.

The above-described operation can be performed repeatedly until the radiation detector 110 or the radiation emitter 210 arrives at the scanning position, or until the radiation emitter 210 rotates to the irradiation angle, in operations 435 and 436.

If the radiation detector 110 or the radiation emitter 210 arrives at the scanning position, or if the radiation emitter 210 rotates to the rotation angle, the at least one of the display 321, the sound output unit 322, and the light of the user interface 320 can output a signal corresponding to the scanning start position and/or the irradiation angle to the outside, in operation 437. In other words, if the m-th position of the radiation detector 110 or the radiation emitter 210 is identical to the scanning position, or if the m-th rotation angle of the radiation emitter 210 is identical to the irradiation angle, the at least one of the display 321, the sound output unit 322, and the light of the user interface 320 can continue to output a signal of a pattern corresponding to the m-th position or the m-th rotation angle. The signal output by the at least one of the display 321, the sound output unit 322, and the light of the user interface 320 can be interrupted according to a user's selection or a predetermined setting. In this case, the predetermined setting can include a setting about the elapse of a predetermined time period.

A method of controlling the radiographic imaging apparatus according the above-described form can be implemented in the form of a program executed by a variety of computer means. The program can include program instructions, data files, and data structures as itself or a combination therewith. The program can be designed or manufactured by using higher level code executed by the computer by using an interpreter, as well as by using a machine code that is produced by a compiler. In addition, the program can be particularly designed to implement the control method of the above mentioned image acquisition apparatus or can be implemented by using various functions or definition that are well-known and available to a group of ordinary skill in the computer software field.

Programs for implementing the method of controlling the radiographic imaging apparatus can be recorded on a recording medium readable by a computer. The recording medium readable by a computer can include various types of hardware devices capable of storing a particular program executed in response to a call from a computer, e.g. magnetic disk storage media such as a hard disk or a floppy disk, optical media such as a magnetic tape, a compact disc (CD) or a DVD, magneto-optical media such as a floptical disk, and semiconductor memory devices such as ROM, RAM, or flash memory.

According to the radiographic imaging apparatus, the radiation irradiation apparatus, the radiation detection apparatus, and the control method of controlling the radiographic imaging apparatus, the radiation emitter and/or the radiation detector can move to an appropriate position for stitching scanning so that the radiographic imaging apparatus can acquire an appropriate radiation image for stitching.

According to the radiographic imaging apparatus, the radiation irradiation apparatus, the radiation detection apparatus, and the control method of controlling the radiographic imaging apparatus, the radiation emitter can be inclined smoothly to an appropriate direction for stitching scanning without performing unnecessary manipulations so that a more appropriate image for stitching can be acquired.

According to the radiographic imaging apparatus, the radiation irradiation apparatus, the radiation detection apparatus, and the control method of controlling the radiographic imaging apparatus, the position and/or direction of the radiation detector and/or the radiation emitter can be set without a user's manual manipulation, resulting in an improvement of a user convenience.

According to the radiographic imaging apparatus, the radiation irradiation apparatus, the radiation detection apparatus, and the control method of controlling the radiographic imaging apparatus, it is possible to guide a user to locate the radiation emitter or the radiation detector at an accurate, appropriate position so as to reduce a time taken for the user to manipulate the radiographic imaging apparatus, resulting in an improvement of convenience and immediacy of radiation scanning.

Hereinbefore a variety of forms of the radiographic imaging apparatus, the radiation irradiation apparatus, and the method of controlling the radiographic imaging apparatus are described, but is not limited thereto. A variety of forms which is implementable by those skilled in the art by correcting and modifying based on the above mentioned form may correspond to the above the radiographic imaging apparatus, the radiation irradiation apparatus, and the method of controlling the radiographic imaging apparatus. For example, when the above-mentioned techniques is executed in a different order from the above-mentioned method, and/or the above-mentioned components such as system, structure, device and circuit is coupled or combined in a manner different from the above-mentioned method or is replaced or substituted by other components or equivalents, the same or the similar result as the above-mentioned the radiographic imaging apparatus, the radiation irradiation apparatus, and the method of controlling the radiographic imaging apparatus may be achieved and those may correspond to an example of the above-mentioned the radiographic imaging apparatus, the radiation irradiation apparatus, and the method of controlling the radiographic imaging apparatus.

Although the present disclosure has been described with an exemplary embodiment, various changes and modifications may be suggested to one skilled in the art. It is intended that the present disclosure encompass such changes and modifications as fall within the scope of the appended claims.

## Claims

1. A radiographic imaging apparatus comprising:
a radiation detector;
a first support configured to enable the radiation detector to move in at least one direction of a up direction and a down direction;
a first brake configured to stop the radiation detector at a plurality of positions; and
an image processor configured to synthesize a plurality of electrical signals output by the radiation detector located at each position of the plurality of positions and to generate at least one radiation image.

2. The radiographic imaging apparatus according to claim 1, wherein the radiation detector moves in the down direction along the first support by a weight of the radiation detector.

3. The radiographic imaging apparatus according to claim 1, further comprising a first weight adder configured to apply an additional weight to the radiation detector.

4. The radiographic imaging apparatus according to claim 3, wherein the first weight adder comprises at least one weight and a weight installing member in which the weight is installed.

5. The radiographic imaging apparatus according to claim 4, wherein the weight installing member comprises at least one of a first weight installing member on which the weight is hung, a second weight installing member into which the weight is inserted, and a third weight installing member with which the weight is coupled by magnetism.

6. The radiographic imaging apparatus according to claim 3, wherein the radiation detector moves in the down direction along the first support by the additional weight.

7. The radiographic imaging apparatus according to claim 1, further comprising:
a second weight adder; and
a detector moving member configured to enable the radiation detector to move in the up direction along the first support by an additional weight applied by the second weight adder.

8. The radiographic imaging apparatus according to claim 7, wherein the detector moving member comprises a rotary body, and further comprises a rotation angle detector configured to sense an amount of rotation of the rotary body.

9. The radiographic imaging apparatus according to claim 1, further comprising a position detector configured to detect a position of the radiation detector when the radiation detector moves.

10. The radiographic imaging apparatus according to claim 9, wherein the first brake stops the radiation detector at the plurality of positions according to the position of the radiation detector detected by the position detector.

11. The radiographic imaging apparatus according to claim 9, further comprising a user interface configured to output a signal according to a comparison between the position of the radiation detector detected by the position detector and a scanning position of the radiation detector.

12. The radiographic imaging apparatus according to claim 1, wherein the first support extends or is shortened such that the radiation detector is movable in at least one direction of the up direction and the down direction.

13. The radiographic imaging apparatus according to claim 1, further comprising a radiation emitter configured to irradiate radiation toward the radiation detector.

14. The radiographic imaging apparatus according to claim 1, further comprising:
a movable pulley connected to the radiation detector;
a plurality of fixing pulleys configured to rotate to move the movable pulley in the up direction or in the down direction; and
a plurality of brakes configured to stop the plurality of fixing pulleys independently or dependently.

15. A method of controlling a radiographic imaging apparatus, comprising:
disposing a radiation detector at a first position;
moving the radiation detector in a up direction or in a down direction by an additional weight applied by a weight adder, when a brake is released;
detecting a position of the radiation detector;
operating the brake according to the position of the radiation detector; and
stopping the radiation detector at a second position by the operation of the brake.
